# EUROPEAN PATENT SPECIFICATION

(11) **EP 2 155 867 B1**
(45) Date of publication and mention of the grant of the patent: **14.10.2015**
(21) Application number: 08747632.1
(22) Date of filing: 05.05.2008
(51) Int. Cl.: C12N 9/16, C12N 9/50, C12N 9/52, C11D 3/386

(54) **STABLE ENZYMATIC PERACID GENERATING SYSTEMS**
STABILE ENZYMATISCHE PERSÄURE-ERZEUGENDE SYSTEME
SYSTÈMES DE GÉNÉRATION DE PERACIDE ENZYMATIQUE STABLES

(30) Priority: 10.05.2007 US 917252 P
(43) Date of publication of application: 24.02.2010
(73) Proprietor: Danisco US Inc., Palo Alto, CA 94304 (US)
(72) Inventor: BARNETT, Christopher C., Palo Alto, California 94304 (US)
(74) Representative: Casley, Christopher Stuart
(86) International application number: PCT/US2008/062633
(87) International publication number: WO 2008/140988

(56) References cited:
- WO-A-2005/056782
- WO-A-2006/119060
- WO-A-2007/044666
- WO-A-2007/044667
- WO-A-2007/133263
- CARBONI-OERLEMANS C ET AL: "Hydrolase-catalysed synthesis of peroxycarboxylic acids: Biocatalytic promiscuity for practical applications" JOURNAL OF BIOTECHNOLOGY, ELSEVIER SCIENCE PUBLISHERS, AMSTERDAM, NL, vol. 126, no. 2, 1 November 2006 (2006-11-01), pages 140-151, XP002460370 ISSN: 0168-1656
- NATTERMANN H ET AL: "Efficient killing of anthrax spores using aqueous and alcoholic peracetic acid solutions" BUNDESGESUNDHEITSBLATT GESUNDHEITSFORSCH.GESUNDHEITSSCHUTZ, SPRINGER, BERLIN, DE, vol. 48, no. 8, 1 August 2005 (2005-08-01), pages 939-950, XP019319123 ISSN: 1437-1588

## Description

### CROSS-REFERENCE TO RELATED APPLICATIONS

This application claims the benefit of U.S. Provisional Application No. 60/917,252, filed on May 10, 2007.

### FIELD OF THE INVENTION

The present invention relates to stable compositions comprising a perhydrolase enzyme, a hydrogen peroxide source, and an ester substrate that efficiently generate aqueous peracid solutions suitable for use in decontamination involving a wide variety of chemical and biological warfare materials, as well as for general surface cleaning and decontamination.

### BACKGROUND OF THE INVENTION

Peracids are widely accepted as a decontamination/disinfection agent. Most notably, peracetic acid (also known as "peroxyacetic acid" or "PAA") is employed widely, *inter alia*, for sterilization of items used in the food industry and in hospitals, as well as for decontamination of biohazards. PAA also can be used as a bleaching chemical in cleaning compositions and industrial processes such as bleaching of pulp. PAA poses relatively few disposal problems after use because it decomposes to compounds which are readily degraded in sewage treatment plants. It has a broad spectrum of activity against microorganisms, and is effective even at low temperatures.

A number of non-enzymatic, chemical or electrochemical methods for preparing aqueous PAA solutions have been described in the prior art. See *e.g.,* U.S. Patent Nos. 6,677,477, 6,211,237, 6,171,551, 5,350,563, and 4,137,256, each of which is hereby incorporated by reference herein.

The typical commercially available aqueous PAA solutions contain about 15% by weight PAA, about 14% by weight hydrogen peroxide, and 28% by weight acetic acid. The use of PAA solutions of this type causes, on account of their unpleasant corrosive and fire-accelerating properties, problems with regard to handling, storage, materials and transport. Thus, PAA has a large chemical "footprint" - meaning that safe production and use of this chemical requires an inordinate amount of energy and resources.

Additionally, the higher ratio of acetic acid to PAA in these commercial solutions exacerbates problems related to the corrosion of stainless steel containers during the cleaning process. A lower ratio of acetic acid would reduce the need to further treat *(e.g.,* passivate) steel containers following cleaning with PAA.

Some of these problems could be attenuated by the development of enzymatic systems for the *in situ* generation of peracids in aqueous solution. Enzymatic peracid generating systems provide numerous significant advantages due to the non-caustic characteristics of the water-based biochemical solvents, which allow users to safely use the system without fear of injury to themselves, their equipment, or the environment. In addition, there is a reduced logistical burden, as the enzyme systems are easily transported, easy to use, and require the use of much less water than traditional decontamination methods. Additionally, by-products of enzymatic peracid generating systems typically are biodegradable. For example, PAA decomposes spontaneously to acetic or propionic acid.

PCT/US06/47022, filed December 8, 2006 (which is hereby incorporated by reference herein) discloses enzymes that efficiently generate aqueous peracid solution in sufficient quantity for use in a range of decontaminating and sanitizing applications. In some embodiments, PCT/US06/47022 discloses an acyl transferase enzyme from *Mycobacterium smegmatis* and variants that generate aqueous PAA solution when the enzyme is added to water along with percarbonate and propylene glycol diacetate (PGDA).

Water soluble containers provide a convenient means for delivering pre-measured compositions into an aqueous solution. Such containers have been described for use with cleaning compositions in a number of U.S. patents. For example, U.S. Pat. No. 5,783,541 teaches a dishwashing composition disposed in a water soluble film, wherein the water soluble film is coated with a water dissolvable glue. U.S. Pat. No. 6,037,319, which teaches a water soluble sachet containing a cleaning composition containing an alcohol and hexylene glycol. U.S. Pat. No. 6,465,413 discloses laundering and dishwashing products contained in a water soluble film sachet, wherein the formulation comprises a granulated percarbonate compound mixed with and encapsulated in an encapsulating blend comprising sulfate, carboxy methyl cellulose and nonionic surfactant. U.S. Pat. No. 6,492,312 discloses a water soluble sachet comprising such a dishwashing composition along with a discrete particle that enhances cleaning in a dishwashing machine. US 20030199415 A1, teaches a cleaning system comprising a water soluble sachet containing a concentrate of a liquid cleaning composition having excellent foam collapse properties and excellent grease cutting property.

U.S. Pat. No. 7,052,520 discloses a water-permeable, but not water-soluble, article referred to as a "sachet" or "bio-bag", for use in an enzymatic fabric cleaning. This "bio-bag" does not dissolve in water but rather contains the enzyme-producing micro-organisms in such a manner that they cannot disperse from it into the wash water.

Notwithstanding the above developments in the art, there remains a need for an enzymatic system for generating peracids that exhibits greater stability, greater ease of use, while lessening the storage, transport and overall chemical issues created by the use of this class of compounds in commercial decontamination applications.

### SUMMARY OF THE INVENTION

The present invention provides stable compositions, systems, and kits that enzymatically generate peracid in aqueous solution, and which can be used in a wide range of decontamination methods and applications, as defined in the claims.

Disclosed herein are stable compositions useful for generating peracid in aqueous solution, wherein said composition comprises: an enzyme with perhydrolase activity, a hydrogen peroxide source, and an ester substrate. These three active ingredients are activated by addition to water resulting in the generation of an aqueous peracid solution. It is a surprising result of the present invention that the compositions according to the claims are highly stable prior to activation (*i.e.,* addition to water), wherein stability corresponds to the composition's ability to generate a peracid upon addition to water in an amount effective for use in sanitizing, disinfecting and/or decontaminating items. That is, the stable compositions of present invention retain their ability to generate peracid upon addition to water (*i.e*., peracid generating activity) for at least 7 days, about 14 days, about 30 days, about 60 days, or longer.

In some embodiments, the stable compositions include additional active ingredients such as additional enzymes, surfactants, detergent formulations, and/or cleaning compositions. However, in one preferred embodiment, the composition includes three active ingredients: an acyl transferase enzyme, the hydrogen peroxide source, and the ester substrate; as defined in claim 1.

In one preferred embodiment, the stable composition comprises the acyl transferase enzyme from *M. smegmatis* (MsAcT), sodium percarbonate, and propylene glycol diacetate (PGDA), wherein the enzyme and percarbonate are solids suspended in the liquid PGDA.

WO 2005/056782 describes methods and compositions comprising at least one perhydrolase enzyme for cleaning and other applications. WO 2007/133263 describes an enzyme system that efficiently generates peracetic acid for use in decontamination applications. WO 2007/044667 describes enzymatic production of peracids from carboxylic acid ester substrates using non-heme haloperoxidases. WO 2006/119060 describes production of peracids using perhydrolytic enzymes and WO 2007/044666 describes enzymatic production of peracids using lactobacilli having perhydrolysis activity.

In some embodiments, the perhydrolase enzyme comprises the amino acid sequence set forth in SEQ ID NO:1 or a variant or homologue thereof. In one embodiment, the perhydrolase enzyme is the S54V variant of SEQ ID NO:1.

In one embodiment, the composition comprises from about 0.001% to about 1% by weight MsAcT, from about 35% to about 45% by weight sodium percarbonate, and from about 65% to about 55% by weight propylene glycol diacetate. Upon addition to water, the stable composition is activated and commences generating peracetic acid (PAA). The weight percent of PAA produced in water does not decrease significantly despite prior storage of the composition at ambient temperature for at least 7 days, about 14 days, about 30 days, about 60 days, or longer.

In preferred embodiments, the resulting peracid aqueous solutions generated by the stable compositions are at least about 0.08%, 0.16%, 0.24%, 0.32%, 0.40%, or greater percentage by weight peracid. In embodiments of the stable composition that generate PAA, the resulting PAA aqueous solutions generated by the stable compositions are at least about 0.08%, 0.16%, 0.24%, 0.32%, 0.40%, or greater percentage by weight PAA. In one preferred embodiment, the PAA aqueous solution is at least about 0.16% PAA by weight.

Additionally, the ratio of peracid to acid produced by the enzymatic activity of the stable composition added to water is at least about 2:1, 4:1, 5:1, 10:1, 20:1, or even greater. In embodiments of the stable composition that generate PAA, the ratio of PAA to acetic acid generated upon addition to water is at least about 2:1, 4:1, 5:1, 10:1, 20:1, or even greater.

The present invention provides systems and kits for generating peracids in aqueous solution that based on the stable composition embodiments. Thus, in one embodiment the invention provides a system for generating peracetic acid in aqueous solution comprising any of the stable compositions of the present invention in a water soluble container. In some embodiments, the container is formed from a water soluble polymer, preferably a polyvinyl alcohol polymer. Generally, the water soluble container can be in any form, preferably a sachet, ampoule, capsule or sphere. In one preferred embodiment, the water soluble container is a sachet comprising a polyvinyl alcohol film.

In another embodiment, the present invention provides a kit for decontamination comprising the packaged combination of: (a) a pre-measured amount of any of the stable compositions of the present invention in a sealed container, wherein the amount of the stable composition generates an aqueous solution of at least about 0.16% peracetic acid by weight upon addition to a set volume of water, and (b) instructions for use of the stable composition. In one embodiment, the sealed container of the kit is large enough to hold at least twice said set volume of water. In another embodiment, the kit further comprises the stable composition packaged within a water soluble container that is packaged within said sealed container. In one preferred embodiment, the water soluble container is a sachet made of a polyvinyl alcohol film. In an alternative embodiment, the packaged combination of the kit further comprises the set volume of water in a second sealed container, and wherein the water is sterilized.

Generally, the stable compositions of the present invention, and the systems and kits incorporating the stable compositions, can be used in various methods for preparing aqueous solutions for decontaminating, disinfecting, and/or sanitizing items. Thus, the present invention also provides methods for preparing a decontamination solution comprising adding the stable composition useful for generating peracetic acid to water and mixing for at least about 30 minutes, 20 minutes, 15 minutes, 10 minutes, or even fewer minutes.

In some embodiments, the method for decontamination comprises: (a) providing a stable composition according to the claims, comprising an enzyme with perhydrolase activity, wherein said activity comprises a perhydrolysis to hydrolysis ratio of at least 2:1; a hydrogen peroxide source; and an ester substrate; and (b) adding said composition to water and mixing for at least 20 minutes, thereby generating an aqueous solution of at least about 0.16% peracetic acid by weight, and a pH less than about 9.0; and (c) exposing an item comprising a contaminant to said solution. In one embodiment, the solution of the stable composition includes no other ingredients capable of buffering the pH.

In one preferred embodiment, the method for decontamination of the present invention comprises: (a) providing a stable composition according to the claims, comprising an acyl transferase enzyme, a hydrogen peroxide source, and propylene glycol diacetate; (b) adding said composition to water and mixing for at least 20 minutes, thereby generating an aqueous solution of at least about 0.16% peracetic acid by weight; and (c) exposing an item comprising a contaminant to said solution. In some embodiments of the method for preparing a solution, the stable composition provided comprises from about 0.001% to about 1% by weight MsAcT, from about 35% to about 45% by weight sodium percarbonate, and from about 65% to about 55% by weight propylene glycol diacetate.

In one preferred embodiment of the methods, the stable composition is contained in a water soluble container and the container is added to the water. In one preferred embodiment, the water soluble container is a sachet, ampoule, capsule or sphere, formed from a water soluble polymer, preferably a polyvinyl alcohol polymer.

In various embodiments, the methods of the invention are useful against a wide range of contaminants including toxins selected from the group consisting of botulinum toxin, anthracis toxin, ricin, scombroid toxin, ciguatoxin, tetrodotoxin, mycotoxins, and any combination thereof; and pathogens selected from the group consisting of bacteria, viruses, fungi, parasites, prions, and any combination thereof. In one embodiment, the methods of the invention are useful against biofilms, including those formed by pathogens including but not limited to: *Pseitdomonas aeruginosa, Staphylococcus aureus* (SRWC-10943), and *Listeria monocytogenes* (ATCC 19112).

In various embodiments, the methods of the invention are useful for decontaminating and/or sanitizing a wide range of contaminated items including hard surfaces, fabrics, food, feed, apparel, rugs, carpets, textiles, medical instruments, veterinary instruments, pharmaceutical processing equipment, and biotechnology processing equipment. In one preferred embodiment, PAA solutions generated using the stable compositions of the present invention may be used to sanitize stainless steel equipment, including large reactors, used in pharmaceutical and biotechnology processes.

### BRIEF DESCRIPTION OF THE DRAWINGS

Figure 1 provides a graph showing the enzymatic generation of PAA from hydrogen peroxide or percarbonate.
Figure 2 provides a graph showing the generation of PAA from glucose and propyleneglycol diacetate.
Figure 3 provides a graph showing the generation of PAA at three different temperatures (21°C, 40°C, and 60°C).
Figure 4 provides a graph showing the ability of the acetyl transferase enzyme to produce concentrated PAA.

### DETAILED DESCRIPTION OF THE INVENTION

### I. Overview

The present invention provides stable compositions, systems, kits, that enzymatically generate peracids in aqueous solution, and which can be used in a wide range of decontamination methods and applications. The present invention is based in part on the surprising discovery that compositions comprising a perhydrolase enzyme, such as acyl transferase, a hydrogen peroxide source, and an ester substrate, such as propylene glycol diacetate, are extremely stable prior to use. Indeed, compositions of these three components can be stable for at least 7 days, about 14 days, about 30 days, about 60 days, or longer, wherein stability corresponds to the ability to generate an effective amount of peracid upon addition to water.

Peracids (*e.g.,* peracetic acid) are well-characterized decontamination agents that are accepted and approved for use in a wide range of applications. *In-situ* enzymatic peracetic acid generation in water, which occurs with a preferred embodiment of the stable composition, systems and kits of the present invention, is desirable as it is much safer than reactive chemical generation in solvents and requires much less volume. Thus, the present invention provides a stable composition that is convenient, simple to use, and highly effective for a wide range decontamination methods and techniques. For example, the stable compositions of the present invention may be used in decontaminating chemical and biological warfare materials, as well as for general surface cleaning and decontamination

In one preferred embodiment, the stable composition comprises the acyl transferase enzyme, MsAcT from *M. smegmatis,* and sodium percarbonate as hydrogen peroxide source. In one embodiment, the composition comprises from about 0.001% to about 1% by weight MsAcT, from about 35% to about 45% by weight sodium percarbonate, and from about 65% to about 55% by weight propylene glycol diacetate.

The stable composition of the present provides numerous advantages over currently used methods that utilize peracid for cleaning, disinfection and/or decontamination. Significantly, the composition is a stable "All-In-One" composition, wherein all of the ingredients are formulated together (*e.g.,* in a single container or package). Consequently, usage of the composition only requires adding the contents of a single container to water. Additionally, the present invention also provides the stable composition in a sealed water soluble container, which allows the further convenience of not having to even open a container. Instead, the composition in the water soluble container is added to water, thereby providing an aqueous solution convenient for decontamination use.

### II. Definitions

Unless defined otherwise herein, all technical and scientific terms used herein have the same meaning as commonly understood by one of ordinary skill in the art to which this invention pertains. For example, Singleton and Sainsbury, Dictionary of Microbiology and Molecular Biology, 2d Ed., John Wiley and Sons, NY (1994); and Hale and Marham, The Harper Collins Dictionary of Biology, Harper Perennial, NY (1991) provide those of skill in the art with a general dictionaries of many of the terms used in the invention. Although any methods and materials similar or equivalent to those described herein find use in the practice of the present invention, the preferred methods and materials are described herein. Accordingly, the terms defined immediately below are more fully described by reference to the Specification as a whole. Also, as used herein, the singular terms "a," "an," and "the" include the plural reference unless the context clearly indicates otherwise. Unless otherwise indicated, nucleic acids are written left to right in 5' to 3' orientation; amino acid sequences are written left to right in amino to carboxy orientation, respectively. It is to be understood that this invention is not limited to the particular methodology, protocols, and reagents described, in the description below, as these may vary, depending upon the context in which they are used by those of skill in the art.

It is intended that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

As used herein, the term "enzyme" refers to any protein that catalyzes a chemical reaction. The catalytic function of an enzyme constitutes its "activity" or "enzymatic activity." An enzyme typically is classified according to the type of catalytic function it carries out, *e.g*., hydrolysis of peptide bonds.

As used herein, the term "substrate" refers to a substance (*e.g.,* a chemical compound) on which an enzyme performs its catalytic activity to generate a product.

A "perhydrolase" refers to an enzyme that is capable of catalyzing a perhydrolysis reaction that results in the production of a sufficiently high amount of peracid suitable for use in an application such as cleaning, bleaching, disinfection, or sterilization. Generally, a perhydrolase enzyme used in methods described herein exhibits a high perhydrolysis to hydrolysis ratio. In some embodiments, the perhydrolase comprises, consists of, or consists essentially of the *Mycobacterium smegmatis* perhydrolase amino acid sequence set forth in SEQ ID NO:1, or a variant or homolog thereof. In some embodiments, the perhydrolase enzyme comprises acyl transferase activity and catalyzes an aqueous acyl transfer reaction.

A "peracid" is an organic acid of the formula RC(=O)OOH.

"Perhydrolysis" or "perhydrolyze" refers to an enzymatic reaction that produces a peracid. In some embodiments, a peracid is produced by perhydrolysis of an ester substrate of the formula R₁C(=O)OR₂, where R₁ and R₂ are the same or different organic moieties, in the presence of hydrogen peroxide (H₂O₂).

The phrase "source of hydrogen peroxide" includes hydrogen peroxide as well as the components of a system that can spontaneously or enzymatically produce hydrogen peroxide as a reaction product.

The phrase "perhydrolysis to hydrolysis ratio" refers to the ratio of the amount of enzymatically produced peracid to the amount of enzymatically produced acid by a perhydrolase enzyme from an ester substrate under defined conditions and within a defined time.

As used herein, the term "acyl" refers to an organic acid group, which is the residue of a carboxylic acid after removal of a hydroxyl (-OH) group (*e.g.,* ethanoyl chloride, CH₃CO-Cl, is the acyl chloride formed from ethanoic acid, CH₃CO-OH). The name of an individual acyl group is general formed by replacing the "-ic" of the acid by "-yl."

As used herein, the term "acylation" refers to a chemical transformation in which an acyl (RCO-) group is substituted into a molecule, generally for an active hydrogen of an -OH group.

As used herein, the term "transferase" refers to an enzyme that catalyzes the transfer of a functional group from one substrate to another substrate.

As used herein, the term "enzymatic conversion" refers to the modification of a substrate or intermediate to a product, by contacting the substrate or intermediate with an enzyme. In some embodiments, contact is made by directly exposing the substrate or intermediate to the appropriate enzyme. In other embodiments, contacting comprises exposing the substrate or intermediate to an organism that expresses and/or excretes the enzyme, and/or metabolizes the desired substrate and/or intermediate to the desired intermediate and/or end-product, respectively.

As used herein, "effective amount of enzyme" refers to the quantity of enzyme necessary to achieve the activity required in the specific application (*e.g.,* production of peracetic acid by acyl transferase for use in decontamination). Such effective amounts are readily ascertained by one of ordinary skill in the art and are based on many factors, such as the particular enzyme variant used, the specific composition, the method of decontamination, the item to be decontaminated, and the like.

As used herein, the term "stability" in reference to a substance (*e.g.,* an enzyme) or composition refers to its ability to maintain a certain level of functional activity over a period of time under certain environmental conditions. Furthermore, the term "stability" can be used in a number of more specific contexts referring to the particular environmental condition that is of interest. For example, "thermal stability" as used herein refers to the ability of a substance or composition to maintain its function (*i.e.,* not degrade) at increased temperature. A substantial change in stability is evidenced by at least about a 5% or greater increase or decrease (in most embodiments, it is preferably an increase) in the half-life of the functional activity being assayed, as compared to the activity present in the absence of the selected environmental conditions.

As used herein, the term "chemical stability" as used in reference to an enzyme refers to the stability of the enzyme in the presence of chemicals that adversely affect its activity. In some embodiments, such chemicals include, but are not limited to hydrogen peroxide, peracids, anionic detergents, cationic detergents, non-ionic detergents, chelants, etc. However, it is not intended that the present invention be limited to any particular chemical stability level nor range of chemical stability, beyond what is stated in the claims.

As used herein, "pH stability" refers to the ability of a substance (*e.g.,* an enzyme) or composition to function at a particular pH. Stability at various pHs can be measured either by standard procedures known to those in the art and/or by the methods described herein. A substantial change in pH stability is evidenced by at least about 5% or greater increase or decrease (in most embodiments, it is preferably an increase) in the half-life of the functional activity, as compared to the activity at the optimum pH. It is not intended that the present invention be limited to any pH stability level nor pH range.

As used herein, "oxidative stability" refers to the ability of a substance (*e.g*., an enzyme) or composition to function under oxidative conditions, *e.g*., in the presence of an oxidizing chemical.

As used herein, "oxidizing chemical" refers to a chemical that has the capability of bleaching. The oxidizing chemical is present at an amount, pH and temperature suitable for bleaching. The term includes, but is not limited to hydrogen peroxide and peracids.

As used herein, the terms "purified" and "isolated" refer to the removal of contaminants from a sample. For example, an acyl transferase is purified by removal of contaminating proteins and other compounds within a solution or preparation that are not acyl transferases.

As used herein, the term "contaminant" refers to any substance which by its contact or association with another substance, material, or item makes it undesirable, impure, and/or unfit for use.

As used herein, the term "a contaminated item" or "item in need of decontamination" refers to any item or thing in contact or associated with a contaminant and/or which needs to be decontaminated. It is not intended that the item be limited to any particular thing or type of item. For example, in some embodiments, the item is a hard surface, while in other embodiments, the item is an article of clothing. In yet additional embodiments, the item is a textile. In yet further embodiments, the item is used in the medical and/or veterinary fields. In some preferred embodiments, the item is a surgical instrument. In further embodiments, the item is used in transportation (*e.g.,* roads, runways, railways, trains, cars, planes, ships, etc.). In further embodiments, the term is used in reference to food and/or feedstuffs, including but not limited to meat, meat by-products, fish, seafood, vegetables, fruits, dairy products, grains, baking products, silage, hays, forage, etc. Indeed, it is intended that the term encompass any item that is suitable for decontamination using the methods and compositions provided herein.

As used herein, the term "decontamination" refers to the removal of substantially all or all contaminants from a contaminated item. In some preferred embodiments, decontamination encompasses disinfection, while in other embodiments, the term encompasses sterilization. However, it is not intended that the term be limited to these embodiments, as the term is intended to encompass the removal of inanimate contaminants, as well as microbial contamination. (*e.g.,* bacterial, fungal, viral, prisons, etc.).

As used herein, the term "disinfecting" refers to the removal of contaminants from the surfaces, as well as the inhibition or killing of microbes on the surfaces of items. It is not intended that the present invention be limited to any particular surface, item, or contaminant(s) or microbes to be removed.

As used herein, the term "sterilizing" refers to the killing of all microbial organisms on a surface.

As used herein, the term "sporicidal" refers to the killing of microbial spores, including but not limited to fungal and bacterial spores. The term encompasses compositions that are effective in preventing germination of spores, as well as those compositions that render spores completely non-viable.

As used herein, the terms "bactericidal," "fungicidal," and "viricidal" refer to compositions that kill bacteria, fungi, and viruses, respectively. The term "microbiocidal" refers to compositions that inhibit the growth and/or replication of any microorganisms, including but not limited to bacteria, fungi, viruses, protozoa, rickettsia, etc.

As used herein, the terms "bacteriostatic," "fungistatic," and "virostatic" refer to compositions that inhibit the growth and/or replication of bacteria, fungi, and viruses, respectively. The term "microbiostatic" refers to compositions that inhibit the growth and/or replication of any microorganisms, including but not limited to bacteria, fungi, viruses, protozoa, rickettsia, etc.

As used herein, the term "cleaning composition" refers to compositions that find use in the removal of undesired compounds from items to be cleaned, such as fabric, dishes, contact lenses, other solid substrates, hair (shampoos), skin (soaps and creams), teeth (mouthwashes, toothpastes) etc. The term further refers to any composition that is suited for cleaning, bleaching, disinfecting, and/or sterilizing any object and/or surface. It is intended that the term includes, but is not limited to detergent compositions (*e.g.,* liquid and/or solid laundry detergents and fine fabric detergents; hard surface cleaning formulations, such as for glass, wood, ceramic and metal counter tops and windows; carpet cleaners; oven cleaners; fabric fresheners; fabric softeners; and textile and laundry pre-spotters, as well as dish detergents). The term further encompasses any materials/compounds selected for the particular type of cleaning composition desired and the form of the product (*e.g.,* liquid, gel, granule, or spray composition), as long as the composition is compatible with the acyl transferase, hydrogen peroxide source, PGDA, and any other enzyme(s) or substance used in the composition. The specific selection of cleaning composition materials is readily made by considering the surface, item or fabric to be cleaned, and the desired form of the composition for the cleaning conditions during use. Indeed, the term "cleaning composition" as used herein, includes unless otherwise indicated, granular or powder-form all-purpose or heavy-duty washing agents, especially cleaning detergents; liquid, gel or paste-form all-purpose washing agents, especially the so-called heavy-duty liquid (HDL) types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, mouthwashes, denture cleaners, car or carpet shampoos, bathroom cleaners; hair shampoos and hair-rinses; shower gels and foam baths and metal cleaners; as well as cleaning auxiliaries such as bleach additives and "stain-stick" or pre-treat types.

As used herein, the terms "detergent composition" and "detergent formulation" are used in reference to mixtures which are intended for use in a wash medium for the cleaning of soiled objects. In some preferred embodiments, the term is used in reference to laundering fabrics and/or garments (*e.g.,* "laundry detergents"). In alternative embodiments, the term refers to other detergents, such as those used to clean dishes, cutlery, etc. (*e.g.,* "dishwashing detergents"). It is not intended that the present invention be limited to any particular detergent formulation or composition. Indeed, it is intended that in addition to a perhydrolase enzyme, *e.g*., an acyl transferase, the term encompasses detergents that contain surfactants, transferase(s), hydrolytic enzymes, oxido reductases, builders, bleaching agents, bleach activators, bluing agents and fluorescent dyes, caking inhibitors, masking agents, enzyme activators, antioxidants, and solubilizers.

As used herein, the term "enzyme compatible," when used in the context of cleaning composition materials means that the materials do not reduce the enzymatic activity to such an extent that the relevant enzyme is not effective as desired during normal use situations.

As used herein, "protein" refers to any composition comprised of amino acids and recognized as a protein by those of skill in the art. The terms "protein," "peptide" and polypeptide are used interchangeably herein. Wherein a peptide is a portion of a protein, those skilled in the art understand the use of the term in context. The terms "wild-type" and "native" are used to refer to proteins found in nature. In some embodiments, the wild-type protein's sequence is the starting point of a protein engineering project.

As used herein, the terms "related protein" or "homologous protein" refer to proteins that are functionally and/or structurally similar (*i.e.,* have similar action and/or structure). It is intended that the term encompass the same or similar enzyme(s) (*i.e.,* in terms of structure and function) obtained from different species. It is not intended that the present invention be limited to related proteins from any particular source(s) or proteins related evolutionarily. In addition, the term related or homologous proteins encompasses tertiary structural homologs and primary sequence homologs. Thus, the terms include proteins with "variant" or "mutant" sequences relative to the wild-type sequence.

As used herein, the term "derivative" refers to a protein which is derived from a parent protein by addition of one or more amino acids to either or both of the C- and N-terminal end(s), substitution of one or more amino acids at one or a number of different sites in the amino acid sequence, and/or deletion of one or more amino acids at either or both C- and N- terminal end(s) and/or at one or more sites in the amino acid sequence, and/or insertion of one or more amino acids at one or more sites in the amino acid sequence. The preparation of a protein derivative is often achieved by modifying a DNA sequence that encodes a native protein, transformation of the modified DNA sequence into a suitable host, and expression of the modified DNA sequence to produce the derivative protein.

Related (and derivative) proteins encompass "variant" proteins. Variant proteins differ from a parent protein and/or from one another by a small number of amino acid residues. In some embodiments, the number of different amino acid residues is any of about 1, 2, 3, 4, 5, 10, 20, 25, 30, 35, 40, 45, or 50. In some embodiments, variants differ by about 1 to about 10 amino acids.

In some embodiments, related proteins, such as variant proteins, comprise any of at least about 35%, 40%, 45%, 50%, 55%, 60%, 65%, 70%, 75%, 80%, 85%, 90%, 95%, 97%, 98%, 99%, or 99.5% amino acid sequence identity.

As used herein, the term "analogous sequence" refers to a polypeptide sequence within a protein that provides a similar function, tertiary structure, and/or conserved residues with respect to a reference protein. For example, in epitope regions that contain an alpha helix or a beta sheet structure, replacement amino acid(s) in an analogous sequence maintain the same structural element. In some embodiments, analogous sequences are provided that result in a variant enzyme exhibiting a similar or improved function with respect to the parent protein from which the variant is derived.

As used herein, "homologous protein" refers to a protein *(e.g.,* a perhydrolase enzyme) that has similar function (*e.g.,* enzymatic activity) and/or structure as a reference protein (*e.g*., a perhydrolase enzyme from a different source). Homologs may be from evolutionarily related or unrelated species. In some embodiments, a homolog has a quaternary, tertiary and/or primary structure similar to that of a reference protein, thereby potentially allowing for replacement of a segment or fragment in the reference protein with an analogous segment or fragment from the homolog, with reduced disruptiveness of structure and/or function of the reference protein in comparison with replacement of the segment or fragment with a sequence from a non-homologous protein.

As used herein, "wild-type," "native," and "naturally-occurring" proteins are those found in nature. The terms "wild-type sequence" refers to an amino acid or nucleic acid sequence that is found in nature or naturally occurring. In some embodiments, a wild-type sequence is the starting point of a protein engineering project, for example, production of variant proteins.

### III. Enzymatic Generation of Peracid in Aqueous Solution

The stable compositions of the present invention comprise at least one enzyme as a component (*i.e.,* ingredient). The enzymes used in the present invention must have significant perhydrolase activity - *i.e.,* a catalytic activity that results in the formation of high amounts of peracid suitable for applications such as cleaning, bleaching, and disinfecting. The use of enzymes with perhydrolase activity for peracid generation is described in WO 05/056782.

Typically, enzymatic perhydrolase activity is accompanied by hydrolase activity. Indeed, as discussed below, many hydrolase enzymes also act as perhydrolases in the presence of hydrogen peroxide. In particularly preferred embodiments, the enzymes of the present invention have very high ratio of perhydrolase to hydrolase activity, and thereby produce a high ratio of peracid relative to acid products (*e.g.,* ratio of peracid to acid of at least about 2:1, 5:1, 10:1, or greater). These high perhydrolysis to hydrolysis ratios of these distinct enzymes makes these enzymes suitable for use in a very wide variety of applications.

In addition to having significant perhydrolase activity, the enzymes useful in the stable compositions of the present invention exhibit relatively low activity in dry or substantially water free conditions. In some embodiments, the enzyme component of the stable composition has less than about 1%, 0.5%, 0.2%, or less than about 0.1% activity (perhydrolase or hydrolase) when there is less about 1% water by weight present in the composition. In preferred embodiments, the enzyme exhibits less than about 0.1% activity when there is about 2% water by weight present or less.

In some embodiments, the perhydrolase enzyme is naturally-occurring (*i.e.,* a perhydrolase enzyme encoded by a genome of a cell). In some embodiments, the perhydrolase enzyme comprises, consists of, or consists essentially of an amino acid sequence that is at least about 80%, 85%, 90%, 95%, 97%, 98%, 99%, or 99.5% identical to the amino acid sequence of a naturally-occurring perhydrolase enzyme.

In some embodiments, the perhydrolase enzyme has a perhydrolysis:hydrolysis ratio of at least 1.

### 1. Acyl transferase enzymes - MsAcT from M. smegmatis

In one embodiment, the present invention provides a stable composition comprising an acyl transferase capable of the enzymatic conversion of hydrogen peroxide and ester substrate to peracid in aqueous solution, as defined in the claims. In preferred embodiments, the stable compositions of the present invention use acyl transferase enzymes that produce very high perhydrolysis to hydrolysis ratios. High perhydrolysis to hydrolysis ratios means that these enzymes produce higher yields of peracid, making them more effective in generating decontamination solutions.

An acyl transferase with a favorable perhydrolysis to hydrolysis ratio that makes it particularly well-suited for the present invention is the *M. smegmatis* acyl transferase (MsAcT). For example, MsAcT when used to enzymatically convert PGDA and hydrogen peroxide from percarbonate generates PAA product in at least 5:1 excess over acetic acid. Because the MsAcT produces a high ratio of PAA to acetic acid, the pH of the resulting aqueous peracid solution is able to remain at relatively low pH, less than about pH 10, about pH 9.0, about pH 8.5, about pH 8.0, or about pH 7.5. Such low pH PAA (or other peracid) aqueous solutions are considered non-corrosive (or at least substantially less corrosive) to metals than typical commercial PAA solutions which contain high levels of acetic acid.

Additionally, the MsAcT is essentially inactive in the absence of water. The MsAcT enzyme requires a significant amount of water present *(i.e.,* relatively high degree of hydration) in order to exhibit perhydrolase or hydrolase activity. Typically, MsAcT and it variants exhibit less than about 1%, 0.5%, 0.2%, or less than about 0.1% activity (perhydrolase or hydrolases) when there is less about 1% water by weight present in the composition that includes the enzyme.

The wild-type *M. smegmatis* acyl transferase is described in WO 05/056782, which is hereby incorporated by reference herein in its entirety. PCT/US06/47022, filed December 8, 2006, which also is incorporated by reference herein, discloses a number of MsAcT variants including S54V-MsAcT, and the use of the wild-type and variants to generate aqueous peracid solution when the enzyme is added to water along with a hydrogen peroxide source and an ester substrate (*e.g.,* propylene glycol diacetate (PGDA)).

In some embodiments, the perhydrolase enzyme is a naturally occurring *M*. *smegmatis* perhydrolase enzyme. In some embodiments, a perhydrolase enzyme comprises, consists of, or consists essentially of the amino acid sequence set forth in SEQ ID NO:1 or a variant or homologue thereof. In some embodiments, a perhydrolase enzyme comprises, consists of, or consists essentially of an amino acid sequence that is at least about 80%, 85%, 90%, 95%, 97%, 98%, 99%, or 99.5% identical to the amino acid sequence set forth in SEQ ID NO:1.

The amino acid sequence of *M. smegmatis* perhydrolase is shown below:

MAKRILCFGDSLTWGWVPVEDGAPTERFAPDVRWTGVLAQQLGADFEVIEEGLSA
RTTNIDDPTDPRLNGASYLPSCLATHLPLDLVIIMLGTNDTKAYFRRTPLDIALGMS
VLVTQVLTSAGGVGTTYPAPKVLVVSPPPLAPMPHPWFQLIFEGGEQKTTELARVY
SALASFMKVPFFDAGSVISTDGVDGIHFTEANNRDLGVALAEQVRSLL (SEQ ID
NO:1)

The corresponding polynucleotide sequence encoding *M. smegmatis* perhydrolase is:

5’-ATGGCCAAGCGAATTCTGTGTTTCGGTGATTCCCTGACCTGGGGCTGGGTCC
CCGTCGAAGACGGGGCACCCACCGAGCGGTTCGCCCCCGACGTGCGCTGGACC
GGTGTGCTGGCCCAGCAGCTCGGAGCGGACTTCGAGGTGATCGAGGAGGGACT
GAGCGCGCGCACCACCAACATCGACGACCCCACCGATCCGCGGCTCAACGGCG
CGAGCTACCTGCCGTCGTGCCTCGCGACGCACCTGCCGCTCGACCTGGTGATCA
TCATGCTGGGCACCAACGACACCAAGGCCTACTTCCGGCGCACCCCGCTCGACA
TCGCGCTGGGCATGTCGGTGCTCGTCACGCAGGTGCTCACCAGCGCGGCGGCG
TCGGCACCACGTACCCGGCACCCAAGGTGCTGGTGGTCTCGCCGCCACCGCTGG
CGCCCATGCCGCACCCCTGGTTCCAGTTGATCTTCGAGGGCGGCGAGCAGAAGA
CCACTGAGCTCGCCCGCGTGTACAGCGCGCTCGCGTCGTTCATGAAGGTGCCGT
TCTTCGACGCGGGTTCGGTGATCAGCACCGACGGCGTCGACGGAATCCACTTCA
CCGAGGCCAACAATCGCGATCTCGGGGTGGCCCTCGCGGAACAGGTGCGGAGC
CTGCTGTAA-3’ (SEQ ID NO:2)

### 2. Variant Acyl Transferase Enzymes

It is not intended that the stable compositions of the present invention be limited to including wild type *M. smegmatis* acyl transferase. In alternative embodiments, an acyl transferase can be used that is a homolog or engineered variant of the MsAcT acyl transferase. In further preferred embodiments, a monomeric hydrolase is engineered to produce a monomeric or multimeric enzyme that has better acyl transferase activity than the native monomeric enzyme. In some particularly preferred embodiments, the variant comprises the substitution S54V of MsAcT (referred to herein as "S54V-MsAcT" or the "S54V variant" or "variant S54V").

In other embodiments, the acyl transferase used in the stable composition is one of the variant enzymes or homologs disclosed and described in WO 05/056782.

In some embodiments, the perhydrolase enzyme comprises one or more substitutions at one or more amino acid positions equivalent to position(s) in the *M. smegmatis* perhydrolase amino acid sequence set forth in SEQ ID NO:1. In some embodiments, the perhydrolase enzyme comprises any one or any combination of substitutions of amino acids selected from M1, K3, R4, I5, L6, C7, D10, S11, L12, T13, W14, W16, G15, V17, P18, V19, D21, G22, A23, P24, T25, E26, R27, F28, A29, P30, D31, V32, R33, W34, T35, G36, L38, Q40, Q41, D45, L42, G43, A44, F46, E47, V48, I49, E50, E51, G52, L53, S54, A55, R56, T57, T58, N59, I60, D61, D62, P63, T64, D65, P66, R67, L68, N69, G70, A71, S72, Y73, S76, C77, L78, A79, T80, L82, P83, L84, D85, L86, V87, N94, D95, T96, K97, Y99F100, R101, R102, P104, L105, D106, I107, A108, L109, G110, M111, S112, V113, L114, V115, T116, Q117, V118, L119, T120, S121, A122, G124, V125, G126, T127, T128, Y129, P146, P148, W149, F150, I153, F154, I194, and F196.

In some embodiments, the perhydrolase enzyme comprises one or more of the following substitutions at one or more amino acid positions equivalent to position(s) in the *M. smegmatis* perhydrolase amino acid sequence set forth in SEQ ID NO:1: L12C, Q, or G; T25S, G, or P; L53H, Q, G, or S; S54V, L A, P, T, or R; A55G or T; R67T, Q, N, G, E, L, or F; K97R; V125S, G, R, A, or P; F154Y; F196G.

In some embodiments, the perhydrolase enzyme comprises a combination of amino acid substitutions at amino acid positions equivalent to amino acid positions in the *M. smegmatis* perhydrolase amino acid sequence set forth in SEQ ID NO:1: L12I S54V; L12M S54T; L12T S54V; L12Q T25S S54V; L53H S54V; S54P V125R; S54V V125G; S54V F196G; S54V K97R V125G; or A55G R67T K97R V125G.

Several methods are known in the art that are suitable for generating variants of the acyl transferase enzymes of the present invention, including but not limited to site-saturation mutagenesis, scanning mutagenesis, insertional mutagenesis, random mutagenesis, site-directed mutagenesis, and directed-evolution, as well as various other recombinatorial approaches.

In some embodiments, acyl transferases useful with the present invention include engineered variants within the SGNH-hydrolase family of proteins. In some preferred embodiments, the engineered proteins comprise at least one or a combination of the following conserved residues: L6, W14, W34, L38, R56, D62, L74, L78, H81, P83, M90, K97, G110, L114, L135, F180, G205. In alternative embodiments, these engineered proteins comprise the GDSL-GRTT and/or ARTT motifs. In further embodiments, the enzymes are multimers, including but not limited to dimers, octamers, and tetramers. In yet additional preferred embodiments, the engineered proteins exhibit a perhydrolysis to hydrolysis ratio that is greater than about 1:1, 2:1, 5:1, or even 10:1.

An amino acid residue of an acyl transferase is equivalent to a residue of *M. smegmatis* acyl transferase if it is either homologous *(i.e.,* having a corresponding position in either the primary and/or tertiary structure) or analogous to a specific residue or portion of that residue in *M. smegmntis* acyl transferase *(i.e.,* having the same or similar functional capacity to combine, react, and/or chemically interact).

In some embodiments, in order to establish homology to primary structure, the amino acid sequence of an acyl transferase is directly compared to the *M. smegmatis* acyl transferase primary sequence and particularly to a set of residues known to be invariant in all acyl transferases for which sequence is known. After aligning the conserved residues, allowing for necessary insertions and deletions in order to maintain alignment *(i.e.,* avoiding the elimination of conserved residues through arbitrary deletion and insertion), the residues equivalent to particular amino acids in the primary sequence of *M. smegmatis* acyl transferase are defined. In preferred embodiments, alignment of conserved residues conserves 100% of such residues. However, alignment of greater than 75% or as little as 50% of conserved residues is also adequate to define equivalent residues. In preferred embodiments, conservation of the catalytic serine and histidine residues are maintained.

Conserved residues are used to define the corresponding equivalent amino acid residues of *M. smegmatis* acyl transferase in other acyl transferases *(e.g.,* acyl transferases from other *Mycobacterium* species, as well as any other organisms).

In some embodiments, the following residues of *M. smegmatis* acyl transferase are modified: Cys7, Asp10, Ser11, Leu12, Thr13, Trp14, Trp16, Pro24, Thr25, Leu53, Ser54, Ala55, Thr64, Asp65, Arg67, Cys77, Thr91, Asn94, Asp95, Tyr99, Val125, Pro138, Leu140, Pro146, Pro148, Trp149, The150, Ile153, Phe154, Thr159, Thr186, Ile192, Ile194, and Phe196. However, it is not intended that the present invention be limited to sequence that are modified at these positions. Indeed, it is intended that the present invention encompass various modifications and combinations of modifications.

In some embodiments, some of the residues identified for substitution, insertion or deletion are conserved residues whereas others are not. The acyl transferase mutants of the present invention include various mutants, including those encoded by nucleic acid that comprises a signal sequence. In some embodiments of acyl transferase mutants that are encoded by such a sequence are secreted by an expression host. In some further embodiments, the nucleic acid sequence comprises a homolog having a secretion signal.

Characterization of wild-type and mutant proteins is accomplished via any means suitable and is preferably based on the assessment of properties of interest. For example, pH and/or temperature, as well as detergent and /or oxidative stability is/are determined in some embodiments of the present invention. Indeed, it is contemplated that enzymes having various degrees of stability in one or more of these characteristics (pH, temperature, proteolytic stability, detergent stability, and/or oxidative stability) will find use. In still other embodiments, acyl transferases with low peracid degradation activity are selected.

### 3. Other Enzymes

Although acyl transferase from *M. smegmatis* (MsAcT) is used in one preferred embodiment of the stable compositions, any perhydrolase enzyme obtained from any source which in the presence of hydrogen peroxide converts an ester substrate into mostly peracid may be used in the present invention. In preferred embodiments, the enzyme exhibit a perhydrolysis to hydrolysis ratio that is greater than about 2:1, 5:1, or even 10:1. Consequently, the ratio of peracid to acid product is greater than about 2:1, 5:1, or even 10:1, and the pH of the resulting aqueous peracid solution is able to remain relatively low, *e.g*., less than about pH 10, about pH 9.0, about pH 8.5, about pH 8.0, or about pH 7.5.

In addition to the acyl transferase enzymes, various hydrolases have perhydrolase activity that makes them useful as enzymes in the compositions of the present invention. Thus, hydrolases useful in the present invention include but are not limited to: carboxylate ester hydrolase, thioester hydrolase, phosphate monoester hydrolase, and phosphate diester hydrolase which act on ester bonds; a thioether hydrolase which acts on ether bonds; and α-amino-acyl-peptide hydrolase, peptidyl-amino acid hydrolase, acyl-amino acid hydrolase, dipeptide hydrolase, and peptidyl-peptide hydrolase which act on peptide bonds. Such hydrolase(s) find use alone or in combination with MsAcT or another perhydrolase. Preferable among them are carboxylate ester hydrolase, and peptidyl-peptide hydrolase.

Other suitable hydrolases include: (1) proteases belonging to the peptidyl-peptide hydrolase class (*e.g*., pepsin, pepsin B, rennin, trypsin, chymotrypsin A, chymotrypsin B, elastase, enterokinase, cathepsin C, papain, chymopapain, ficin, thrombin, fibrinolysin, renin, subtilisin. aspergillopeptidase A, collagenase, clostridiopeptidase B, kallikrein, gastrisin, cathepsin D, bromelin, keratinase, chymotrypsin C, pepsin C, aspergillopeptidase B, urokinase, carboxypeptidase A and B, and aminopeptidase); (2) carboxylate ester hydrolase including carboxyl esterase, lipase, pectin esterase, and chlorophyllase; and (3) enzymes having high perhydrolysis to hydrolysis ratios. Especially effective among them are lipases, as well as esterases that exhibit high perhydrolysis to hydrolysis ratios, as well as protein engineered esterases, cutinases, and lipases, using the primary, secondary, tertiary, and/or quaternary structural features of the perhydrolases of the present invention.

Additionally, it is contemplated that the stable compositions of the present invention may comprise additional enzymes. It is contemplated that by using combinations of enzymes, there will be a concurrent reduction in the amount of chemicals needed. The additional enzymes include but are not limited to: microbial cell wall-degrading and glycoprotein-degrading enzymes, lysozyme, hemicellulases, peroxidases, proteases, cellulases, xylanases, lipases, phospholipases, esterases, cutinases, pectinases, keratinases, reductases, oxidases, phenoloxidases, lipoxygenases, ligninases, pullulanases, tannases, pentosanases, malanases, ß-glucanases, arabinosidases, hyaluronidase, chondroitinase, laccase, endoglucanases, PNGases, amylases, etc., as well as mixtures thereof. In some embodiments, enzyme stabilizers may also be included in the stable composition.

It is not intended that the present invention be limited to any specific enzyme for the generation of hydrogen peroxide, as any enzyme that generates H₂O₂ and acid with a suitable substrate finds use in the methods of the present invention. For example, lactate oxidases from *Lactobacillus* species which are known to create H₂O₂ from lactic acid and oxygen find use with the present invention. Indeed, one advantage of the methods of the present invention is that the generation of acid reduces the pH of a basic solution to the pH range in which the peracid is most effective in bleaching *(i.e.,* at or below the pKa).

Other enzymes (*e.g.,* alcohol oxidase, ethylene glycol oxidase, glycerol oxidase, amino acid oxidase, etc.) that can generate hydrogen peroxide also find use with ester substrates in combination with the acyl transferase or other perhydrolase enzymes of the present invention to generate peracids. Enzymes that generate acid from substrates without the generation of hydrogen peroxide also find use in the present invention. Examples of such enzymes include, but are not limited to proteases. Thus, as described herein, the present invention provides definite advantages over the currently used methods and compositions for decontaminant formulation and use, as well as various other applications.

In addition, oxidases find use in the present invention, including carbohydrate oxidases selected from the group consisting of aldose oxidase (IUPAC classification EC 1.1.3.9), galactose oxidase (IUPAC classification EC 1.1.3.9), cellobiose oxidase

(IUPAC classification EC1.1.3.25), pyranose oxidase (IUPAC classification EC1.1.3.10), sorbose oxidase (IUPAC classification EC1.1.3.11) and/or hexose oxidase (IUPAC classification EC1.1.3.5), glucose oxidase (IUPAC classification EC1.1.3.4) and mixtures thereof.

Indeed, it is contemplated that any suitable oxidase can be used in the present invention that follows the equation:

Enzyme + reduced substrate → oxidized substrate + H₂O₂.

### 4. Hydrogen Peroxide Source

The present invention provides a stable composition comprising a hydrogen peroxide source. In some embodiments, the hydrogen peroxide source is a solid compound that generates hydrogen peroxide upon addition to water. Such compounds include adducts of hydrogen peroxide with various inorganic or organic compounds, of which the most widely employed is sodium carbonate perhydrate, also referred to as sodium percarbonate.

Inorganic perhydrate salts are one preferred embodiment of hydrogen peroxide source. Examples of inorganic perhydrate salts include perborate, percarbonate, perphosphate, persulfate and persilicate salts. The inorganic perhydrate salts are normally the alkali metal salts.

Other hydrogen peroxide adducts useful in the compositions of the present invention include adducts of hydrogen peroxide with zeolites, or urea hydrogen peroxide.

The hydrogen peroxide source compounds may be included as the crystalline and/or substantially pure solid without additional protection. For certain perhydrate salts however, the preferred executions of such granular compositions utilize a coated form of the material which provides better storage stability for the perhydrate salt in the granular product. Suitable coatings comprise inorganic salts such as alkali metal silicate, carbonate or borate salts or mixtures thereof, or organic materials such as waxes, oils, or fatty soaps.

In some embodiments, the present invention provides a stable composition wherein the hydrogen peroxide source is an enzymatic hydrogen peroxide generation system. In one preferred embodiment, the enzymatic hydrogen peroxide generation system comprises an oxidase and its substrate. Suitable oxidase enzymes include, but are not limited to: glucose oxidase, sorbitol oxidase, hexose oxidase, choline oxidase, alcohol oxidase, glycerol oxidase, cholesterol oxidase, pyranose oxidase, carboxyalcohol oxidase, L-amino acid oxidase, glycine oxidase, pyruvate oxidase, glutamate oxidase, sarcosine oxidase, lysine oxidase, lactate oxidase, vanillyl oxidase, glycolate oxidase, galactose oxidase, uricase, oxalate oxidase, and xanthine oxidase.

In some embodiments, the hydrogen-peroxide generating compounds and enzyme systems can be combined into the stable composition in their substantially pure form. In alternative embodiments, they may be combined with other components such as surfactants, sequestrants, builders, pH regulators, buffers, stabilizers, processing additives or any other known components of washing compositions, sanitising compositions, disinfecting compositions or bleach additives. In one embodiment, the stable composition comprises a detergent.

Other compounds and enzymatic systems useful as hydrogen peroxide sources in the stable compositions of the present invention include but are not limited those described in U.S.S.N. 10/581,014

### 5. Ester Substrates and Peracids

It is not intended that the stable compositions of the present invention be limited to ingredients that generate only peracetic acid. Stable compositions may also be prepared that produce any of a wide range of peracids useful for cleaning, disinfecting, and decontaminating.

Pernonanoic acids, as well as peracids made from long chain fatty acids *(i.e.,* C10-C18) or longer chains, may be generated from All-In-One compositions prepared according to the teachings of the present invention. In some preferred embodiments, the peracid is selected from peracetic acid, perproprionic, perbutanoic, perpentanoic, and perhexanoic acid, and pernonanoic acid.

A stable All-In-One composition for generating a specific peracid compound upon addition to water requires the use of an ester substrate that upon enzymatic conversion results in the desired peracid.

As described herein, the ester substrate is a stable ester of an acid selected from the list consisting of: acetic acid, propionic acid, butanoic acid, pentanoic acid, hexanoic acid, nonanoic acid, formic acid, butyric acid, valeric acid, caproic acid, caprylic acid, decanoic acid, dodecanoic acid, myristic acid, palmitic acid, stearic acid, and oleic acid. In additional embodiments, triacetin, tributyrin, neodol esters, and/or ethoxylated neodol esters serve as ester substrates for peracid formation. Thus, exemplary acetic ester substrates include but are not limited to: methyl acetate, ethyl acetate, propyl acetate, triacetin (1, 3-diacetyloxypropan-2-yl acetate), and propylene glycol diacetate.

In a preferred embodiment, the ester substrate is a stable alcohol ester of an acid the enzymatic conversion of which results in the desired peracid. Thus, in one preferred embodiment, the ester substrate is any poly-alcohol ester compound, *e.g*., a poly-ol diester such as PGDA. In another preferred embodiment, the ester substrate is a diol-diester compound.

A wide range of ester substrates for perhydrolase enzymes (*e.g.,* acyl transferase) useful in the compositions of the present invention are disclosed in WO 05/056782.

The ester substrates useful in the stable compositions disclosed herein may be in either solid or liquid form. Generally, the ester substrate should be dry or substantially free of water that can activate the enzyme component of the composition. In preferred embodiments, the ester substrate includes less about 5%, less than about 1%, less than about 0.5%, less than about 0.1%, or even a lower percentage by weight of water.

In some embodiments, the ester substrate is a stable liquid at room temperature, for example, propylene glycol diacetate (PGDA). In some embodiments, the ester substrate is a stable liquid, in which the enzyme and hydrogen peroxide source components of the stable composition do not dissolve. In preferred embodiments, the ester substrate is substantially free of water, thereby minimizing any activation of the enzyme and/or the hydrogen peroxide source in the composition prior to its use.

### 6. Adjunct Materials and Additional Components

Additional components find use in the formulations of the present invention. Although it is not intended that the formulations of the present invention be so limited, various components are described herein. Indeed, while such components are not essential for the purposes of the present invention, the non-limiting list of adjuncts illustrated hereinafter are suitable for use in the instant compositions and may be desirably incorporated in certain embodiments of the invention, for example to assist or enhance cleaning performance, for treatment of the substrate to be cleaned, or to modify the aesthetics of the cleaning composition as is the case with perfumes, colorants, dyes or the like. It is understood that such adjuncts are in addition to the enzymes of the present invention, hydrogen peroxide and/or hydrogen peroxide source and material comprising an ester moiety. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, deposition aids, dispersants, corrosion inhibitors, additional enzymes, and enzyme stabilizers, catalytic materials, bleach activators, bleach boosters, preformed peracids, polymeric dispersing agents, clay soil removal/anti-redeposition agents, brighteners, suds suppressors, dyes, perfumes, structure elasticizing agents, carriers, hydrotropes, processing aids and/or pigments. In addition to the disclosure below, suitable examples of such other adjuncts and levels of use are found in U.S. Patent Nos. 5,576,282, 6,306,812, and 6,326,348. The aforementioned adjunct ingredients may constitute the balance of the cleaning compositions of the present invention.

In some embodiments, the enzyme system of the present invention further comprises enzymes that remove any residual peracid and/or H₂O₂ after decontamination has been achieved. Such enzymes include but are not limited to catalases and/or hydrolytic enzymes.

Importantly, the present invention provides means for effective cleaning, bleaching, and disinfecting over broad pH and temperature ranges. In some embodiments, the pH range utilized in this generation is 4-12. In some alternative embodiments, the temperature range utilized is between about 5° and about 90°C. The present invention provides advantages over the presently used systems (*See e.g.,* EP Appln. 87-304933.9) in that bleaching is possible at the optimum pH of peracid oxidation, as well as providing bleaching at neutral pH, acidic pHs, and at low temperatures.

### IV. Preparation and Use of Stable Compositions for Generating Peracid Solutions

One key advantage of the stable compositions of the present invention is that they are "All-In-One" formulations where all the ingredients are in one container. These stable All-In-One compositions also have the advantage of easy preparation simply by combining the ingredients (*e.g.,* enzyme, hydrogen peroxide source, and ester substrate) in a suitable vessel or container. The order of mixing the ingredients is not particularly important and generally the various ingredients can be added sequentially.

In some embodiments, all of the ingredients of the composition are solids which are simply combined by mixing. In other embodiments, at least one of the ingredients, typically the ester substrate, is a liquid, and preparation is by mixing the dry solid ingredients into the liquid.

Preferably, the stable composition is prepared using solid forms of enzyme(s) and hydrogen peroxide generating compound(s) and mixing them so they are suspended in a liquid form of substrate ester compound. In one preferred embodiment, the stable composition of the present invention comprises an acyl transferase enzyme, a hydrogen peroxide source, and the liquid ester substrate, PGDA.

In one preferred embodiment, the solid ingredients may be combined in a "dry formulation" (*e.g.,* acyl transferase enzyme + percarbonate) which is then added to a liquid ester substrate so as to create a suspension. *See e.g.,* preparation of the All-In-One composition disclosed herein in Example 9.

In one preferred embodiment, the solid ingredients of the stable composition are used as-is. For example, granules or powder of sodium percarbonate (*i.e*., straight out of a commercially obtained reagent jar) are combined in a container with crude solid or granules of acyl transferase, then added to the liquid PGDA to create a suspension. In alternative embodiments, the solid ingredients can be treated prior to combining with each other, or prior to addition to any liquid phase. Examples of such treatments could include encapsulation of enzyme granules and/or peroxide generating compounds in order to inhibit activity during storage, or effect slower release over time after addition to water.

Generally, the stability of the composition is highly dependent on preventing any enzymatic conversion of the substrate, and/or generation of hydrogen peroxide, prior to use. In embodiments where all of the composition ingredients are in solid form, this is accomplished primarily by keeping the mixed ingredients dry or substantially water-free *(i.e.,* less than about 2%, 1%, or 0.5% water by weight). Even relatively small amounts of water that can allow enzyme activity and/or hydrogen peroxide generation to occur should be avoided in order to maximize stability of the All-In-One composition. Consequently, in some embodiments the stable composition further comprises a dessicant or drying agent (*e.g.,* zeolite compound, molecular sieves, etc.).

In one embodiment, the enzyme(s) is incorporated into the stable composition in the form of granules or a spray-dried coating of enzyme-containing crude extract. In other embodiments, the enzyme(s) used in the composition is substantially purified and in a granular, spray-dried, lyophilized, or otherwise dry solid form. In some embodiments, enzyme granules are used which have been formulated so as to contain an enzyme protecting agent and a dissolution retardant material (*i.e*., material that regulates the dissolution of granules during use).

The present invention can be employed in connection with any number of processes for making dry enzyme granules or dry formulations of hydrogen peroxide source compounds, such as Enzoguard^{®} (Genencor International Inc., Rochester, N.Y.) described in U.S. Pat. No. 5,324,649, (which is hereby incorporated by reference herein), or Savinase granules (Novo Nordisk, Denmark), among others. Other exemplary dry formulation processes for enzymes or other compounds which can be used to make the compositions of the present invention include those disclosed in, U.S. Pat. Nos. 4,106,991. 4,689,297, 5,814,501, 5,879,920, 6,248,706, 6,413,749, 6,534,466, or PCT publications WO 97/12958, WO 99/32613, WO 01/29170, and those techniques described in "Enzymes in Detergency," ed. Jan H. van Ee, et al., Chpt. 15, pgs. 310-312 (Marcel Dekker, Inc., New York, N.Y. (1997))

The amounts of hydrogen peroxide source and ester substrate used in the stable composition depends on the specific compounds used as well as the enzyme and final concentration of peracid. Generally, the enzymatic generation of a peracid requires an equimolar amount of hydrogen peroxide and the corresponding ester substrate compound. Consequently, in one preferred embodiment the weight percentage of each of the hydrogen peroxide source and the substrate depends on the formula weight of the specific compounds and is selected so that there are equimolar amounts of hydrogen peroxide and substrate.

The enzyme is incorporated into the composition as much as required according to the purpose. Generally, enzyme should preferably be incorporated in an amount of 0.00001 to 5 weight percent, and more preferably 0.02 to 3 weight percent. Where crude enzyme is used, the granules are added to the stable composition in such an amount that the purified enzyme is 0.001 to 50 weight percent in the granules. Relatively smaller amounts by weight are used with increasing enzyme purity. The granules are used in an amount of 0.002 to 20 and preferably 0.1 to 10 weight percent. Ultimately, specific amounts of enzyme will depend to some extent on the particular enzyme's empirically determined activity for a particular peracid application.

For example, in one preferred embodiment, the stable composition comprises the acyl transferase enzyme from *M. smegmatis* (MsAcT), sodium percarbonate, and propylene glycol diacetate (PGDA), wherein the enzyme and percarbonate are solids suspended in the liquid PGDA, and whereby upon addition to water, the stable composition is capable of generating at least about 0.16% by weight peracetic acid solution. Because of the high ratio of PAA to acetic acid produced by the composition, an aqueous solution of 0.16% by weight PAA has a pH about 8.6. In some embodiments, longer mixing/reaction times for the composition in water can result in even higher levels of PAA (*e.g*., 0.18% by weight) with lower pH *(e.g.,* about pH 8.0).

In this embodiment, the composition is prepared by mixing a dry formulation of enzyme and percarbonate into the liquid PGDA. The resulting stable composition is a liquid suspension preferably comprising: from about 0.001% to about 1% by weight MsAcT, from about 35% to about 45% by weight sodium percarbonate, and from about 65% to about 55% by weight propylene glycol diacetate. The stability of this liquid suspension is demonstrated by the fact that the weight percent of peracetic acid it produces in a set amount of water does not decrease significantly despite prior storage of the composition at ambient temperature for at least 7 days, about 14 days, about 30 days, about 60 days, or longer.

The use of the stable compositions of the present invention to prepare an aqueous solution is straightforward: simply add the composition to an appropriate amount of water and stir. Generally, only purified *(e.g.,* MilliQ water), distilled, sterilized and/or deionized water is used. Thus, the present invention also provides a method for preparing a peracid solution comprising adding the stable composition to water and mixing for at least about 30 minutes, 20 minutes, 15 minutes, 10 minutes, 5 minutes, or even fewer minutes. In some embodiments, the method of preparation comprises: (a) providing a stable composition comprising an enzyme with perhydrolase activity, wherein said activity comprises a perhydrolysis to hydrolysis ratio of at least 2:1; a hydrogen peroxide source; and an ester substrate; and (b) adding said composition to water and mixing for at least 20 minutes, thereby generating an aqueous solution of at least about 0.16% peracetic acid by weight, and a pH less than about 9.0. In one embodiment, the composition and the water comprise no other ingredients capable of buffering the pH.

In preferred embodiments, the method of preparation comprises: (a) providing a stable composition comprising an acyl transferase enzyme, a hydrogen peroxide source, and propylene glycol diacetate; and (b) adding said composition to water and mixing for at least 20 minutes, thereby generating an aqueous solution of at least about 0.16% peracetic acid by weight.

It is contemplated that in addition to pure water, the stable compositions can be used in various aqueous systems, including those that have a large percentage of water (e.g., more than about 85%, or more than about 95% water), as well as those with lower percentages of water (*e.g.,* less than about 85%). Alternative embodiments are contemplated where the stable composition is added to aqueous buffer solutions, and/or solutions comprising other ingredients such as surfactants, cleaning compositions, or detergent formulations. In such embodiments, the other ingredients included in the aqueous solution can affect the catalytic activity of the peracid generating enzyme. Adjustments to the enzyme concentration, stirring time, temperature, and/or other parameters related to the use of the stable composition are within the knowledge of, or may be determined empirically by, one of ordinary skill in the art.

Generally, the stable compositions of the present invention are intended to be used by adding to water at typical ambient temperatures of about 18°C to about 25°C, or about 20°C to about 22°C. In some applications, however, it may be preferred to use the stable compositions at lower or higher temperatures. The stable compositions are capable of functioning over wide temperature ranges (*e.g.,* from about 5°C to about 90°C; from about 16°C to about °60C; and from about 20°C to about 37°C). Generally, the functional temperature range is dependent on the ability of the enzyme to maintain sufficient activity. Thus, in one embodiment, the present invention contemplates a stable composition comprising wild-type or engineered enzymes with high thermal stability.

Furthermore, the present invention provides systems and kits for generating peracids in aqueous solution that based on the stable composition embodiments. Thus, in one embodiment the invention provides a system for generating peracetic acid in aqueous solution comprising any of the stable compositions of the present invention in a water soluble container. In some embodiments, the container is formed from a water soluble polymer, preferably a polyvinyl alcohol polymer. Generally, the water soluble container can be in any form, preferably a sachet, ampoule, capsule or sphere. In one preferred embodiment, the water soluble container is a sachet comprising a polyvinyl alcohol film.

### V. Water Soluble Containers

Water soluble containers useful for delivering the stable compositions of the present invention can be in the form of a sachet, a capsule, or other blow-molded shapes, an injected molded ampoule, or other injection-molded shapes, or rotationally-molded spheres or capsules are formed from a water soluble thermoplastic resin.

Water soluble plastics which may be useful for forming the container include low molecular weight and/or chemically modified polylactides; such polymers have been produced and sold under the name HEPLON^{™} (Chronopol, Inc.).

In one embodiment, the water soluble containers useful with the present invention comprise melt processable polyvinyl alcohol resins (PVA). Such resins include VINEX^{™}; (Texas Polymer Services, Inc.) and MONOSOL^{™} film (ChrisCraft Film). Any number or combination of PVA resins can be used. Such water soluble polymers are described in *e.g.,* U.S. Patent Nos. 6,956,070, 7,022,656, and 7,067,575, each of which is hereby incorporated by reference herein.

Typical resin properties are:
1. Glass Transition Temperature (°C) = 28 to 38; preferred is 28 to 33.
2. Weight Average Molecular Weight (Mw) = 15,000 to 95,000; preferred is 55,000-65,000.
3. Number Average Molecular Weight (Mn) = 7,500 to 60,000; preferred is 27,000 to 33,000.

In one embodiment, the poly(vinyl) alcohol resin used is MONOSOL^{™} 7030 or MONOSOL^{™} 8630.

Blow molded capsules can be formed from the polyvinyl alcohol resin having a molecular weight of about 50,000 to about 70,000 and a glass transition temperature of about 28 to about 33°C. Pelletized resin and concentrate(s) are fed into an extruder. The extruder into which they are fed has a circular, oval, square or rectangular die and an appropriate mandrel. The molten polymer mass exits the die and assumes the shape of the die/mandrel combination. Air is blown into the interior volume of the extrudate while the extrudate contacts a pair of split molds. The molds control the final shape of the package. While in the mold, the package is filled with the appropriate volume of liquid. The mold quenches the plastic. The liquid is contained within the interior volume of the blow molded package.

An injection molded ampoule or capsule is formed from the poly(vinyl) alcohol resin having a molecular weight of about 50,000 to about 70,000 and a glass transition temperature of about 28 to 38°C. Pelletized resin and concentrate(s) are fed to the throat of a reciprocating screw, injection molding machine. The rotation of the screw pushes the pelletized mass forward while the increasing diameter of the screw compresses the pellets and forces them to contact the machine's heated barrel. The combination of heat, conducted to the pellets by the barrel and frictional heat, generated by the contact of the pellets with the rotating screw, melts the pellets as they are pushed forward. The molten polymer mass collects in front of the screw as the screw rotates and begins to retract to the rear of the machine. At the appropriate time, the screw moves forward forcing the melt through the nozzle at the tip of the machine and into a mold or hot runner system which feeds several molds. The molds control the shape of the finished package. The package may be filled with liquid either while in the mold or after ejection from the mold. The filling port of the package is heat sealed after filling is completed. This process may be conducted either in-line or off-line.

A rotationally molded sphere or capsule is formed from the poly(vinyl) alcohol resin having a molecular weight of about 50,000 to about 70,000 and a glass transition temperature of about 28 to 38°C. Pelletized resin and concentrate are pulverized to an appropriate mesh size, typically 35 mesh. A specific weight of the pulverized resin is fed to a cold mold having the desired shape and volume. The mold is sealed and heated while simultaneously rotating in three directions. The powder melts and coats the entire inside surface of the mold. While continuously rotating, the mold is cooled so that the resin solidifies into a shape which replicates the size and texture of the mold. After rejection of the finished package, the liquid is injected into the hollow package using a heated needle or probe after filling, the injection port of the package is heat sealed.

Other suitable resins useful for water soluble containers of the present invention include polyethylene oxides (*e.g.,* POLYOX^{™}; Union Carbide, Inc.) and cellulose derived water soluble carbohydrates (*e.g.,* METHOCEL^{™}; Dow Chemical, Inc.). Typically, the cellulose derived water soluble polymers are not readily melt processable.

In some embodiments, the water soluble container is a sachet. In one particularly preferred embodiment, the sachet may be formed from PVA film. Such PVA film sachets are commercially available (Solupak, Ltd., UK).

Generally, PVA film sachets useful with the present invention may be commercially obtained *(e.g.,* from Solupak, Ltd., UK), or may be manufactured using polymer film manufacturing techniques well-known in the art. For example, the pelletized, pre-dried, melt processable PVA resin, is fed to a film extruder. The feed material may also contain pre-dried color concentrate which uses a PVA carrier resin. Other additives, similarly prepared, such as antioxidants, UV stabilizers, anti-blocking additives, etc. may also be added to the extruder. The resin and concentrate are melt blended in the extruder. The extruder die may consist of a circular die for producing blown film or a coat hanger die for producing cast film. Circular dies may have rotating die lips and/or mandrels to modify visual appearance and/or properties.

Typical PVA film properties useful for the water-soluble sachets of the present invention include:
1. Tensile strength (125 mil, break, 50% RH) = 4,700 to 5,700 psi.
2. Tensile modulus (125 mil, 50% RH) = 47,000 to 243,000 psi; preferred range is 140,000 to 150,000 psi.
3. Tear resistance (mean) (ASTM-D-199 gm/ml) = 900-1500.
4. Impact strength (mean) (ASTM-D-1709, gm) = 600-1,000.
5. 100% Elongation (mean) (ASTM-D-882, psi) = 300-600.
6. Oxygen transmission (1.5 mil, 0% RH, 1 atm) = 0.0350 to 0.450 cc/100 sq. in./24 h.
7. Oxygen transmission (1.5 mil, 50% RH, 1 atm) = 1.20 to 1.50 cc/100 sq. in./24 h.
8. 100% modulus (mean) (ASTM-D-882, psi) = 1000-3000.
9. Solubility (sec) (MSTM-205,75°F.) disintegration=1-15; dissolution =10-30.

The extruded film is slit to the appropriate width and wound on cores. Each core holds one reel of film. The reels of slit film are fed to either a vertical form, fill, seal machine (VFFS) or a horizontal form, fill, seal machine (HFFS). The Form, Fill, Seal machine (FFS) makes the appropriate sachet shape (cylinder, square, pillow, oval, etc.) from the film and seals the edges longitudinally (machine direction seal). The FFS machine also makes an end seal (transverse direction seal) and fills the appropriate volume of nonaqueous liquid above the initial transverse seal. The FFS machine then applies another end seal. The liquid is contained in the volume between the two end seals.

In one embodiment, the PVA film used is MONOSOL^{™} having a weight average molecular weight range of about 55,000 to 65,000 and a number average molecular weight range of about 27,000 to 33,000.

### VI. Kits

The stable compositions of the invention described herein are particularly amenable to packaging in various kits. In one embodiment, the present invention provides a kit for decontamination comprising a pre-measured amount of any of the stable compositions described herein, wherein the stable composition comprises a perhydrolasae enzyme, a hydrogen peroxide source, and an ester substrate, and wherein the amount of the stable composition generates an aqueous solution of at least about 0.16% peracid by weight upon addition to a set volume of water, in suitable packaging. In some embodiments, the kit further comprises instructions for use of the stable composition in a method for decontamination as described herein. Instructions may be provided in printed form or in the form of an electronic medium such as a floppy disc, CD, or DVD, or in the form of a website address where such instructions may be obtained.

In one preferred embodiment, the stable composition is provided in a water soluble sealed container. In one preferred embodiment, the water soluble container of the kit is in the form of a sachet, a capsule, an ampoule, spheres formed from a water soluble thermoplastic resin, or other injection-molded shapes. In some embodiments, the water soluble sealed container is made of a water soluble polymer material selected from the group consisting of: polyvinyl alcohol, polyethylene oxides, cellulose derived water soluble carbohydrates, and polyactides. In one preferred embodiment, the water soluble container of the kit is a sachet made of a polyvinyl alcohol film.

In one embodiment of the kit, kit further comprises a mixing container (or vessel) with a volume large enough to hold at least twice said set volume of water. In this embodiment, the mixing container may be a disposable bucket or beaker, optionally sterilized, that optionally contains a magnetic stir bar in the container along with the stable composition.

In one embodiment, a sealed container comprising the stable composition is packaged inside the mixing container of the kit. Thus, usage of such a kit requires merely adding water to the mixing container with stirring.

In another embodiment, the kit further comprises a set volume of water in a sealed container. In one embodiment, the water in the sealed container is sterilized. In one embodiment, the kit comprises the stable composition in a first sealed container and water in a second sealed container.

In another embodiment, the kit includes a stirring device *(e.g.,* a spoon, or similar implement) and/or a magnetic stir bar packaged in the kit, but outside the sealed container. In other embodiments, the kit further comprises a separate package containing a composition for neutralizing the peracid solution after use.

### VII. Methods and Uses for Sanitizing, Disinfecting and/or Decontaminating

The stable compositions of the present invention (and related systems and kits incorporating these compositions) can be used in a range of methods for decontaminating, disinfecting, and/or sanitizing items.

In some embodiments, the method for decontamination of the present invention comprises: (a) providing a stable composition according to the claims comprising an enzyme with perhydrolase activity, wherein said activity comprises a perhydrolysis to hydrolysis ratio of at least 2:1; a hydrogen peroxide source; and an ester substrate; and (b) adding said composition to water and mixing for at least 20 minutes, thereby generating an aqueous solution of at least about 0.16% peracetic acid by weight, and a pH less than about 9.0; and (c) exposing an item comprising a contaminant to said solution.

In one preferred embodiment, the method for decontamination of the present invention comprises: (a) providing a stable composition comprising an acyl transferase enzyme, a hydrogen peroxide source, and propylene glycol diacetate; (b) adding said composition to water and mixing for at least 20 minutes, thereby generating an aqueous solution of at least about 0.16% peracetic acid by weight; and (c) exposing an item comprising a contaminant to said solution. In some embodiments of the method for preparing a solution, the stable composition provided comprises from about 0.001% to about 1% by weight MsAcT, from about 35% to about 45% by weight sodium percarbonate, and from about 65% to about 55% by weight propylene glycol diacetate.

Depending on the specific type of contaminant to be removed, the step of exposing the item to the peracid solution may be performed over a wide range of time scales. For example, in certain sanitizing procedures exposure times as short as about 30 seconds, 1 minute, 5 minutes or 10 minutes may be sufficient. However, in other applications (e.g., removal of biofilms), it may be necessary to expose the item for considerably longer periods of time, such as about 30 minutes, 1 hour, 6 hours, 12 hours, 24 hours, or even longer, in order to achieve adequate level of decontamination.

Similarly, the temperature of the peracid solution during the exposure step may be adjusted depending on the particular type of contaminant. In a preferred embodiment, the exposure temperature is the ambient temperature at which the solution is prepared, i.e., typically about 18-25°C. In other embodiments, higher temperatures may be used to facilitate the decontamination process. Generally, higher temperatures will accelerate the reactivity of the peracid solution thereby accelerating the decontamination process. Thus, in some embodiments, the exposure step may be carried out with the peracid solution at about 30°C. 37°C. 45°C, 50°C, 60°C, 75°C, 90°C or even higher.

In one preferred embodiment of the methods, the stable composition is contained in a water soluble container and the container is added to the water. In one preferred embodiment, the water soluble container is a sachet, ampoule, capsule or sphere, formed from a water soluble polymer, preferably a polyvinyl alcohol polymer.

In various embodiments, the methods of decontamination using the stable compositions are useful against a wide range of contaminants including toxins selected from the group consisting of botulinum toxin, anthracis toxin, ricin, scombroid toxin, ciguatoxin, tetrodotoxin, mycotoxins, and any combination thereof; and pathogens selected from the group consisting of bacteria, viruses, fungi, parasites, prions, and any combination thereof. For example, the methods disclosed herein may be used for decontamination of materials contaminated with materials including but not limited to toxic chemicals, mustard, VX, *B. anthracis* spores, *Y. pestis, F. tularensis*, fungi, and toxins (*e.g.,* botulinum, ricin, mycotoxins, etc.), as well as cells infected with infective virions (e.g., flaviviruses, orthomyxoviruses, paramyxoviruses, arenaviruses, rhabdoviruses, arboviruses, enteroviruses, bunyaviruses, etc.). In some particularly preferred embodiments, the at least one pathogen is selected from *Bacillus spp., B. anthracis, Clostridium spp., C. botulinum, C. perfringens, Listeria spp., Pseudomonas spp., Staphylococcers spp., Streptococcus spp., Salmonella spp., Shigella spp., E. coli, Yersinia spp., Y. pestis, Francisella spp., F. tularensis, Camplyobacter ssp., Vibrio spp., Brucella spp., Cyptosporidicrm spp., Giardia spp., Cyclospora spp.,* and *Trichinella spp.*

A biofilm is a collection of microorganisms embedded in a matrix of extracellular polymeric substances and various organic and inorganic compounds. Although some biofilms may contain a single species of microorganism, typically biofilms comprise not only different species of microorganisms but different types of microorganisms, for example algae, protozoa, bacteria and others. It has been found that one of the characterizing features of biofilms is that the microorganisms therein act cooperatively or synergistically. Empirically it has been found that microorganisms living in a biofilm are better protected from biocides than microorganisms living outside a biofilm. Thus, removal of pathogenic biofilms represents a particularly difficult problem in decontaminating and/or sanitizing equipment.

The peracid solutions generated using the stable compositions of the present invention and the methods of their use are effective at decontaminating biofilms. In one embodiment, the stable composition made be used to generate a peracid solution useful to remove biofilms, including those formed by one or more pathogenic bacteria selected from the group consisting of: *Bacillus spp., B. anthracis, Clostridium spp., C. botulinum, C. perfringens, Listeria spp., Pseudomonas spp., Staphylococcus spp., Streptococcus spp., Salmonella spp., Shigella ssp., E. coli, Yersinia spp., Y. pestis, Francisella spp., F. tularensis, Camplyobacter ssp., Vibrio spp., Brucella spp., Cryptosporidium spp., Giardia spp., Cyclospora spp., Trichinella spp.,* and any combination thereof. In one preferred embodiment, a peracid solution made by the methods of the present invention may be used to decontaminate biofilms selected from group consisting of: *Pseudomonas aeruginosa, Staphylococcus aureus* (SRWC-10943), *Listeria monocytogenes* (ATCC 19112), and any combination thereof.

In one preferred embodiment, pathogenic biofilms comprising bacterial cultures of *Pseudomonas spp., Staphylococcus spp.,* and/or *Listeria spp.,* contaminating stainless steel equipment can be substantially removed (*i.e*., ~ 500-1000-fold reduction) by exposure to a 0.16% by weight PAA solution (generated from the stable composition) at 45°C for 45 minutes.

In various embodiments, the methods of decontamination using the stable compositions are useful for decontaminating a wide range of contaminated items including hard surfaces, fabrics, food, feed, apparel, rugs, carpets, textiles, medical instruments, veterinary instruments, sanitize, and stainless steel items and equipment, including large reactors, used in pharmaceutical and biotechnology processes.

The peracid solutions generated enzymatically using the stable compositions of the present invention are particularly well-suited for cleaning stainless steel items and equipment because the ratio of peracid to corresponding acid generated in aqueous solution is much higher than found in commercial solutions. For example, a PAA solution generated using the stable composition of S54V-MsAcT, percarbonate, and PGDA, will have a ratio of PAA to acetic acid of approximately 10:1. Commercial PAA solutions typically have more acetic acid than PAA and may even have the reversed ratio (1:10). The increased ratio of PAA to acetic acid reduces, or completely obviates, the need to carry out further passivating treatments of the stainless steel item or equipment following the PAA treatment. Thus, in some embodiments, peracid solutions generated using the stable compositions of the present invention may be used to sanitize stainless steel items and equipment, including large reactors, used in pharmaceutical and biotechnology processes. In preferred embodiments, the peracid solutions may be used to sanitize stainless steel items and equipment in a single-step, without the need for any further treatment of the steel with a passivating agent.

In still further embodiments, the present invention finds use in decontamination of food and/or feed, including but not limited to vegetables, fruits, and other food and/or feed items. Indeed, it is contemplated that the present invention will find use in the surface cleaning of fruits, vegetables, eggs, meats, etc. Indeed, it is intended that the present invention will find use in the food and/or feed industries to remove contaminants from various food and/or feed items. In some particularly preferred embodiments, methods for food and/or feed decontamination set forth by the Food and Drug Administration and/or other food safety entities, as known to those of skill in the art find use with the present invention.

In still further preferred embodiments, the item in need of decontamination is selected from hard surfaces, fabrics, food, feed, apparel, rugs, carpets, textiles, medical instruments, and veterinary instruments. In some particularly preferred embodiments, the food is selected from fruits, vegetables, fish, seafood, and meat. In some still further preferred embodiments, the hard surfaces are selected from household surfaces and industrial surfaces. In some particularly preferred embodiments, the household surfaces are selected from kitchen countertops, sinks, cupboards, cutting boards, tables, shelving, food preparation storage areas, bathroom fixtures, floors, ceilings, walls, and bedroom areas. In some alternative embodiments, the industrial surfaces are selected from food processing areas, feed processing areas, tables, shelving, floors, ceilings, walls, sinks, cutting boards, airplanes, automobiles, trains, and boats.

### EXAMPLES

The following examples are provided in order to demonstrate and further illustrate certain preferred embodiments and aspects of the present invention and are not to be construed as limiting the scope thereof.

In the experimental disclosure which follows, the following abbreviations apply: °C (degrees Centigrade); RT (room temperature); rpm (revolutions per minute); H₂O (water); dH₂O (distilled water); HCl (hydrochloric acid); aa (amino acid); bp (base pair); kb (kilobase pair); kD (kilodaltons); gm (grams); µg and ug (micrograms); mg (milligrams); ng (nanograms); µl and ul (microliters); ml (milliliters); mm (millimeters); nm (nanometers); µm and um (micrometer); M (molar); mM (millimolar); µM and uM (micromolar); U (units); V (volts); MW (molecular weight); sec (seconds); min(s) (minute/minutes); hr(s) (hour/hours); MgCl₂ (magnesium chloride); NaCl (sodium chloride); OD₄₂₀ (optical density at 420 nm); PAGE (polyacrylamide gel electrophoresis); EtOH (ethanol); LB (Luria broth); LA (Luria agar); PBS (phosphate buffered saline [150 mM NaCl, 10 mM sodium phosphate buffer, pH 7.2]); SDS (sodium dodecyl sulfate); Tris (tris(hydroxymethyl)aminomethane); w/v (weight to volume); v/v (volume to volume); wt % (weight percent); PGDA (1,2-propylene glycol diacetate); PAA (peracetic acid); Per (perhydrolase); *per* (perhydrolase gene); Ms (*M. smegmatis*); MsAcT (*M. smegmatis* acyl transferase); S54V-MsAcT or S54V variant (*M. smegmatis* acyl transferase variant comprising the S54V substitution); MS (mass spectroscopy); Dial (Dial Brands, Inc., Scottsdale, AZ); Kemira (Kemira Industrial Chemicals, Helsingborg, Sweden); EM Science (EM Science, Gibbston, NJ); HP (Hewlett-Packard, Palo Alto, CA); ICN (ICN Pharmaceuticals, Inc., Costa Mesa, CA); Dial (Dial, Corp., Scottsdale, AZ); Pierce (Pierce Biotechnology, Rockford, IL); Amicon (Amicon, Inc., Beverly, MA); ATCC (American Type Culture Collection, Manassas, VA); Amersham (Amersham Biosciences, Inc., Piscataway, NJ); Becton Dickinson (Becton Dickinson Labware, Lincoln Park, NJ); BioRad (BioRad, Richmond, CA); Difco (Difco Laboratories, Detroit, MI); GIBCO BRL or Gibco BRL (Life Technologies, Inc., Gaithersburg, MD); MIDI (MIDI Labs, Newark, DE); Sigma or Aldrich (Sigma-Aldrich Inc., St. Louis, MO); Sorvall (Sorvall Instruments, a subsidiary of DuPont Co., Biotechnology Systems, Wilmington, DE); Agilent (Agilent Technologies, Palo Alto, CA); Minolta (Konica Minolta, Ramsey, NJ); and Zeiss (Carl Zeiss, Inc., Thornwood, NY).

### EXAMPLE 1

### Killing Curve for B. subtilis Spores by Peracetic Acid (PAA)

In this Example, experiments conducted to determine the killing curve of peracetic acid (PAA) and PAA in conjunction with detergent (commercially available PUREX® [Dial] was used in this Example) for *B. subtilis* spores. In these experiments, the *B. subtilis* spores were prepared as known in the art (*See e.g.,* Siccardi et al., J. Bacteriol., 121:13-19 [1975]). Assays were carried out in duplicate in 96-well, round bottomed microtiter plates (Costar) with peracetic acid (32 wt % in acetic acid; Aldrich). The PAA was serially diluted in either 50 mM KPO₄ buffer, pH 7.1 ("Buffer"), or in a 1:500 dilution of Purex (original formula; Dial) in the same buffer ("Buffer + Det") in a total volume of 50 µl. The amount of PAA added to the assay was 0, 0.4, 4 or 40 mM. A volume of 5 µl of the spore suspension, containing 10⁹ -10¹⁰ spores, was then added to each well and the assay incubated for 15 min at RT. Ice cold LB (150 µl) (*See e.g.,* Sambrook *et al*., "Molecular Cloning: A Laboratory Manual", Second Edition (Cold Spring Harbor), [1989]) was then added to each well, mixed, and 100 µl transferred to a fresh 96-well plate. Serial dilutions of each solution were made (in a total volume of 100 µl/well). A volume of 5 µl of each dilution was spotted onto LA plates (Sambrook *et al., supra*), and incubated at 37°C for 17-24 h. Colonies were counted and the % spore killing was determined relative to the respective controls (buffer alone or buffer + detergent, without peracid). The results are presented in Table 1, as an average of duplicates from one experiment. However, the experiment was done twice in duplicate with similar results. Based on these results, PAA in the range of about 4 to about 40 mM was determined to be sufficient to kill *B. subtilis* spores in 15 min.

| **Table 1: Killing of *B. subtilis* Spores by PAA** | | | | |
|---|---|---|---|---|
| | **Buffer** | | **Buffer + Detergent** | |
| **[PAA] (mM)** | **Spores/ml** | **% Spore Killing** | **Spores/ml** | **% Spore Killing** |
| 0 | 1.8x10⁹ | 0 | 1.6 x 10⁹ | 0 |
| 0.4 | 2.4x10⁸ | 86.4 | 1.7 x 10⁹ | 0.05 |
| 4 | 1.6x10⁸ | 90 | 2.4 x 10⁸ | 86.4 |
| 40 | 0 | 100 | 0 | 100 |

### EXAMPLE 2

### Enzymatic Generation of PAA

In this Example, three methods for generation of PAA by acyl transferase are described. In one method, at least one acyl transferase (wild-type or variant) is combined with at least one ester substrate, and hydrogen peroxide in a buffer or detergent, with or without one or more surfactants. In an alternative method, at least one acyl transferase (wild-type or variant), at least one ester substrate, and sodium percarbonate (or other source of H₂O₂) are combined in a buffer or detergent, with or without one or more other surfactants. In yet a further method, at least one acyl transferase (wild-type or variant) is combined with glucose oxidase and glucose, in a concentration sufficient to generate an amount of PAA with which to kill spores in buffer or detergent. In some formulations, one or more other surfactants are also included. Other enzymes that generate H₂O₂ also find use in this system, including oxidases, oxidoreductases (*e.g.*, glyerol oxidase or hexose oxidase). In some preferred embodiments, a co-factor independent alcohol oxidase is used.

### Determination of PAA Concentration

In these experiments, methods known in the art were used to determine the concentration of PAA *(See e.g.,* Pinkernell et al., Analyst, 122:567-571 [1997]). In this ABTS assay, 100 µl of the solution to be analyzed was added to 1 ml of 125 mM potassium citrate buffer, pH 5.0, containing 1.0 mM 3-ethylbenzthiazoline-6-sulfonic acid (ABTS) and 50 uM KI, and allowed to incubate at RT for 3 minutes. The absorbance was measured at 420 nm in a HP 8452A Diode Array Spectrophotomer and compared to a standard curve prepared using authentic standard. The enzymatic reactions to form PAA were initiated with addition of enzyme and conducted at RT. Aliquots were withdrawn from the reactions at the indicated times and analyzed for PAA concentration. The sodium percarbonate used in these experiments was obtained from Kemira and the hydrogen peroxide was obtained from EM Science.

### Comparison of PAA Enzymatically Generated from H₂O₂ or Sodium Percarbonate

A solution of 39 mM sodium percarbonate (sodium carbonate peroxyhydrate, Technical grade 85%, yielding 100 mM effective H₂O₂; Kemira) was prepared in 320 mM KPO₄ pH 7.1. After dissolution of the solid percarbonate, the resulting solution had a pH of 7.6. To compare the enzymatic production of PAA from prepared H₂O₂ (32 wt %, Aldrich) or H₂O₂ formed from percarbonate under identical pH conditions, two reactions were prepared. One reaction contained 100 mM H₂O₂ in 320 mM KPO₄ pH 7.6, 100 mM 1,2-propylene glycol diacetate (Aldrich), and 2 ppm variant S54V. The absolute concentration of H₂O₂ was assumed from the value stated on the label and not confirmed by analysis. The second reaction contained 39 mM sodium percarbonate in 320 mM KPO₄, pH 7.1, 100 mM 1,2-propylene glycol diacetate, and 2 ppm S54V. The reactions were initiated by the addition of the enzyme. Samples were withdrawn at the times indicated and the concentration of PAA determined as described in Example 1.

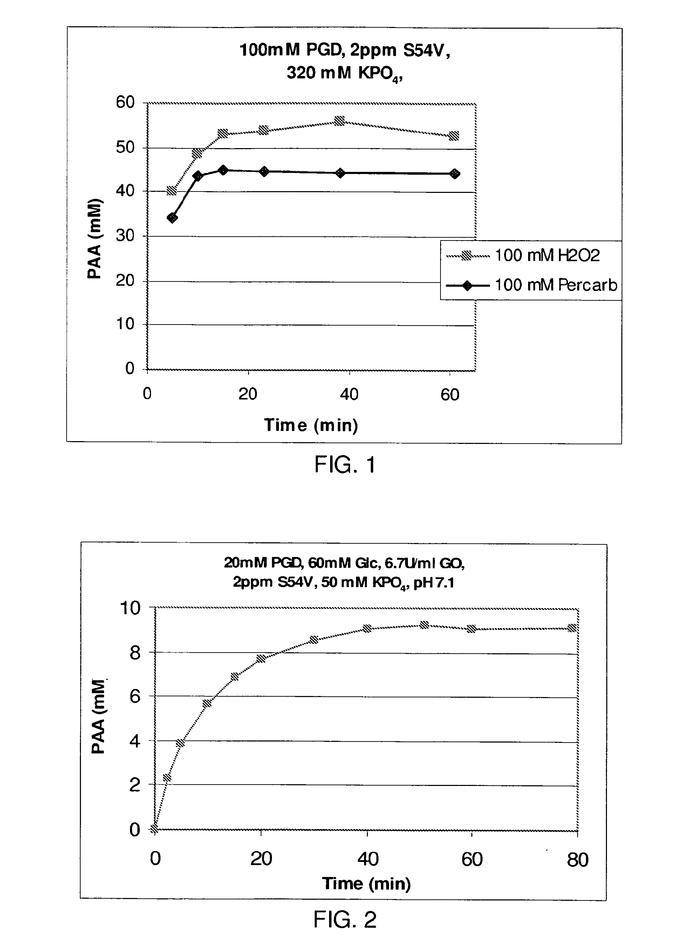

The results for PGDA as substrate are shown in Figure 1. As indicated in Figure 1, the progress of the reaction and final concentration of PAA is similar in both cases.

In order to study the use of different propyl alcohol acetate ester substrates, the generation of PAA was carried out using the same general method described above for PGDA, but using either propyl acetate or triacetin as the ester substrate. It was observed that these other two ester substrates also reacted in the system with S54V-MsAcT to make PAA. The relative conversion rate to PAA for the three ester substrates was observed to be: PGDA > triacetin > propyl acetate.

### EXAMPLE 3

### Enzymatic Generation of PAA Kills B. subtilis Spores

In this Example, experiments conducted to assess the killing ability of enzymatically generated PAA tested with *B. subtilis* spores are described. Based on the results obtained in the experiments described in Examples 1 and 2, a range of 4 to 40 mM PAA was determined to be sufficient to demonstrate killing of spores of *B. subtilis* I-168. In these experiments, spore killing was assessed in buffer, as well as in detergent.

### Spore Killing in Buffer

In this experiment, sodium percarbonate was used as the source of H₂O₂. The final solution contained: 100 mM 1,2-propylene glycol diacetate, 2 ppm S54V variant, 39 mM sodium percarbonate (Technical grade 85%; yielding 100 mM effective H₂O₂) in 320 mM KPO₄ pH 7.1 in a total volume of 800 µl. This mix (yield 40 mM PAA) was serially diluted to give additional mixes that yielded 4.9, 9.9 and 20.5 mM PAA. A mix with only 400 mM KPO₄ pH 7.1 was used to determine total spore counts in the absence of PAA. The mixes were allowed to incubate at room temperature for 3 min. A volume of 180 µl of each of the mixes was then dispensed into duplicate wells of a round-bottomed 96-well plate (Costar) that contained 20 µl of the spore suspension used in Example 1, to yield a total volume of 200 µl in each well. The liquid was gently pipetted 4-5 times to ensure mixing of the components. The mixes were incubated with the spores for a further 15 or 30 minutes at room temperature. At the 15 and 30 minute time points, 20 µl were removed from each of the wells, added to wells in a fresh 96-well plate and serially diluted in LB to 10⁻⁷ in a total volume of 100 µl. A volume of 5 µl from each dilution of each spore mixture was spotted onto an LA plate, allowed to dry and then incubated overnight at 37°C. Also at the 15 and 30 min time points, an appropriate volume was removed from each well and diluted sufficiently in dH₂O to yield a measurable amount of PAA using the ABTS assay on scale with a standard, as described in Example 1. Results of these assays are shown in Table 2. The results of the spore killing are presented as an average of the duplicates.

| **Table 2: Use of MsAcT System to Generate PAA to Kill *B. subtilis* Spores in Buffer** | | | | | | |
|---|---|---|---|---|---|---|
| **[PAA] (mM) (Theoretical)** | **[PAA] Generated (mM) (15 min)** | **Spores/ml 15 min** | **%Spore Killing 15 min** | **[PAA] Generated (mM) (30 min)** | **Spores/ml 30 min** | **%Spore Killing 30 min** |
| 0 | 0 | 1.5 x 10⁹ | 0 | 0 | 1.5 x 10⁹ | 0 |
| 4.9 | 2.2 | 9 x 10⁸ | 40 | 2.4 | 9.4 x 10⁸ | 37 |
| 9.9 | 6.1 | 1 x 10⁸ | 93.3 | 7.1 | 5 x 10⁷ | 96.7 |
| 20.5 | 15 | 4.4 x 10⁴ | 99.997 | 18 | 0 | 100 |
| 39.5 | 35 | 0 | 100 | 41 | 0 | 100 |

### Spore Killing in Detergent.

The experiment was repeated exactly as described except that a 1:500 dilution of Purex in 320 mM KPO₄ pH 7.1. was used in place of the buffer. The results are presented in Table 3 (average of duplicates). Controls included various reaction components in 400 mM KPO₄ pH 7.1 buffer 2 ppm S54V variant, 2 ppm S54V variant with 39 mM percarbonate, 100 mM 1,2-propylene glycol diacetate, 100 mM 1,2-propylene glycol diacetate with 39 mM sodium percarbonate. 39 mM sodium percarbonate. All these treatments gave equivalent levels of spores/ml after a 30 min incubation, except sodium percarbonate alone (1x10⁹ spores/ml vs 5x 10⁹ spores/ml for other controls). This decrease was not seen with sodium percarbonate in combination with other components and was certainly not as dramatic as the killing seen by the mixture of all 3 components at comparable levels (100% killing).

| **Table 3: Use of MsAcT System to Generate PAA to Kill *B. subtilis* Spores in Detergent** | | | | | | |
|---|---|---|---|---|---|---|
| **[PAA] (mM) (Theoretical)** | **[PAA] Generated (mM) (15 min)** | **Spores/ml 15 min** | **%Spore Killing 15 min** | **[PAA] Generated (mM) (30 min)** | **Spores/ml 30 min** | **%Spore Killing 30 min** |
| 0 | 0 | 2x10⁹ | 0 | 0 | 2x10⁹ | 0 |
| 4.9 | 3.7 | 2x10⁹ | 0 | 3.3 | 5x10⁸ | 75 |
| 9.9 | 8.9 | 1.7x10⁹ | 91.5 | 8.1 | 4x10⁷ | 98 |
| 20.5 | 19.6 | 3x10⁵ | 99.99 | 18.9 | 0 | 100 |
| 39.5 | 44.5 | 0 | 100 | 40.4 | 0 | 100 |

### EXAMPLE 4

### Enzymatic Generation of PAA to Kill Trichoderma reesei Spores

In this Example, experiments conducted to assess the killing ability of PAA on T. *reesei* spores are described. *T. reesei* spores were prepared by growing the strain for approximately 4 days on Potato Dextrose (PDA) media at 30°C. When the plate was approximately 75% covered by fungal growth, it was incubated at room temperature for several days until there was confluent growth. The spores were scraped off the plate using a cotton-tipped swab, resuspended in 1 ml of 10% glycerol and frozen at -80°C until used. Prior to use in the spore killing assay, the spore suspension was thawed, the spores pelleted by centrifugation, washed twice with 1 ml dH₂O, and resuspended in 1 ml of dH₂O. The spore killing experiments were carried out as described in Example 3, except that 20 µl of the fungal spore preparation were added to the wells of the 96-well plate instead of the *Bacillus* spores. Also, the mixes were made up such that the amount of PAA generated was 40, 13.3, 4.4 and 1.5 mM. The dilutions of the 15 and 30 minute incubations were plated on PDA media. The actual amount of PAA generated was determined as described in Example 1, at the 15 and 30 minute time points. The results are presented in Table 4. These results indicate that fungal spores were killed by PAA generated by the MsAcT system and at a lower level of PAA than the *B. subtilis* spores.

| **Table 4. Use of MsAcT System to Generate PAA to Kill *Trichoderma reesei* Spores** | | | | | | |
|---|---|---|---|---|---|---|
| **[PAA] Generated (mM) (Theoretical)** | **[PAA] Generated (mM) 15 min** | **Spores/ml 15 min** | **% Spore Killing 15 min** | **[PAA] Generated (mM) 30 min** | **Spores/ml 30 min** | **% Spore Killing 30 min** |
| 0 | 0 | 2x10¹⁰ | 0 | 0 | 2x10⁷ | 0 |
| 1.5 | 0.41 | 2x10³ | 99.99 | 0.3 | 2x10³ | 99.99 |
| 4.4 | 2.1 | 0 | 100 | 2.2 | 0 | 100 |
| 13.3 | 10 | 0 | 100 | 8.5 | 0 | 100 |
| 40 | 42 | 0 | 100 | 38 | 0 | 100 |

### EXAMPLE 5

### PAA Production from Glucose and PGDA

In this Example, experiments to assess the amount of peracetic acid produced from glucose and propyleneglycol diacetate are described. A 15 ml solution was prepared containing 50 mM KPO₄ pH 7.1 with 60 mM glucose, and 20 mM 1,2-propylene glycol diacetate (Aldrich). The solution was continuously sparged with air and stirred at room temperature. The reaction to generate H₂O₂ was initiated by the addition of 100 Units of glucose oxidase (Oxygen HP, Genencor International) and allowed to proceed for 1 hr. A sample was withdrawn and tested for PAA before the addition of 2 ppm S54V variant, to initiate the production of PAA from the formed H₂O₂. The results are presented in Figure 2. Further samples were withdrawn at the times indicated in Figure 2, and the concentration of PAA determined as described above. No PAA was detected before the addition of enzyme and approximately 9.5 mM PAA was produced from the 20 mM 1,2-propylene glycol diacetate and the H₂O₂ produced from the glucose/ glucose oxidase reaction.

### EXAMPLE 6

### Generation of PAA by MsAcT at Different Temperatures

In this Example, experiments were conducted to assess the generation of peracetic acid by MsAcT at different temperatures are described. Such generation provides means to resolve problems associated with storage instability of PAA at various temperatures. In these experiments, MsAcT is used to generate PAA over a range of temperatures from about 20°C to about 60°C. In some experiments, temperatures such as 21°C, 40°C, and 60°C are used.

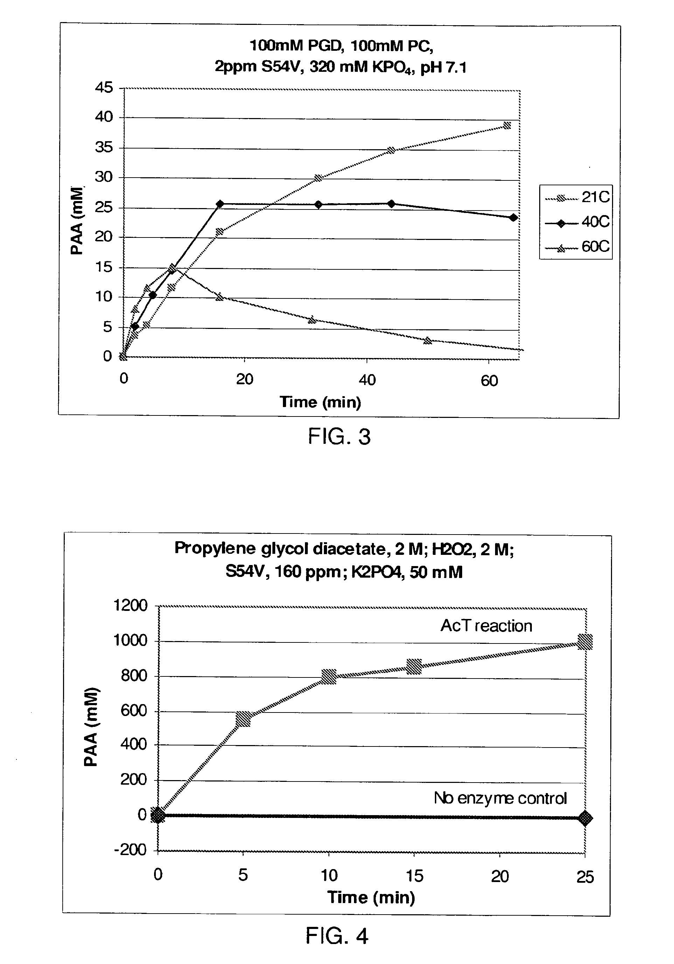

In these experiments, three reactions were prepared, consisting of 320 mM KPO₄ pH 7.1, 100 mM 1,2-propylene glycol diacetate (Aldrich), and 100 mM sodium percarbonate. The reactions were equilibrated at 21°C, 40°C and 60°C, and then initiated by the addition of S54V variant to a final concentration of 2 ppm. The results are presented in Figure 3. Samples were withdrawn at the times indicated in the Figure, and the concentration of PAA determined as described above. These results indicate that the enzyme system is functional at least up to 60°C.

### EXAMPLE 7

### Generation of Concentrated Peracetic Acid by MsAcT

In this Example experiments were conducted to determine the ability of MsAcT to generate a concentrated solution of peracetic acid. These experiments were conducted in order to address the potential benefit of preparing a concentrated peracetic acid solution which is suitable for dosing or dilution into different solutions for use. In this experiment the reaction contained 50 mM KPO₄, 2 M H₂O₂ (EM Science), 2 M 1,2-propylene glycol diacetate (Aldrich), and the S54V variant to a final concentration of 160 ppm. The reaction was vortexed occasionally to mix the reactants, as they were not miscible at this concentration. Mixing was conducted at room temperature. The results are shown in Figure 4. Samples were diluted at the indicated times and the peracetic acid concentration was determined as described above.

### EXAMPLE 8

### An Enzymatic PAA Generating System With High Sporicidal Activity

This example illustrates the effectiveness of a 0.16% by weight peracetic acid (PAA) solution, generated using a three component formulation of the S54V mutant of the acyl transferase enzyme from *M. smegmatis* (S54V-MsAcT), 1,2-propylene glycol diacetate (PGDA) (as ester substrate), and sodium percarbonate (as hydrogen peroxide source), in a number of EPA approved decontamination assays.

### Materials and Methods

Preparation of 250 mL PAA test solution: A dry formulation was made up in 15 mL conical tubes, each containing from about 1.20 g to about 1.30 g sodium percarbonate, and from about 26.0 mg to about 31.9 mg of granules of S54V-MsAcT enzyme. For each dry formulation tube, a separate 15 mL conical tube was prepared containing 1.8 mL of the ester substrate, propylene glycol diacetate (PGDA). One dry formulation tube was packaged with one PGDA tube in a single zip lock bag. The contents of one zip lock bag (*i.e.*, one dry formulation tube and one PGDA tube) constituted one unit for generation of 0.16% PAA upon addition to 250 mL of water.

PAA test solution was prepared by adding the dry formulation from one unit into 250 mL of room temperature sterilized deionized water until fully dissolved. Some of the 250 mL test solution was used to rinse any residual dry formulation from the tube into the solution. Then the tube of PGDA liquid from was added to the stirring 250 mL solution and once again, some of the solution was used to rinse any residual PGDA from the tube into the solution. The PAA test solution was then allowed to stir for 20 minutes. The PAA test solution was homogeneous by visual inspection. It was used within two hours of preparation.

Preparation of 2000 mL PAA test solution: A dry formulation was made up in 15 mL conical tubes, each containing from about 9.6 g to about 9.8 g sodium percarbonate, and from about 216 mg to about 226 mg of granules of S54V-MsAcT enzyme. For each dry formulation sample, a separate 15 mL conical tube was prepared containing 1.8 mL of the ester substrate, propylene glycol diacetate (PGDA). One dry formulation tube was packaged with one PGDA tube in a single zip lock bag. The contents of one zip lock bag it (*i.e.,* one dry formulation tube and one PGDA tube) constituted one unit for generation of 0.16% PAA upon addition to 2000 mL of water.

Preparation of the 2000 mL PAA test solution was the same as described above for the 250 mL test solution except for the larger amounts of dry formulation, PGDA liquid and water.

### Assay 1: Germicidal and Detergent Sanitizing Action

PAA test solutions were prepared according to the 250 mL recipe above. A suspension of either *Staphylococcus aureus* (ATCC 6538) or *Escherichia coli* (ATCC 11229) cells was exposed to the PAA test solution for either 30 seconds or 60 seconds. Following exposure, an aliquot was transferred to the neutralizing subculture medium (Letheen broth + 0.1 % sodium thiosulfate + 0.01% catalase) and assayed for survivors utilizing pour plating techniques and Tryptone glucose extract agar (TGEA). Appropriate numbers control, purity, sterility, viability, and neutralization controls also were performed.

### Assay 2: Inanimate Non-Food Contact Surface Sanitizing Efficacy

PAA test solutions were prepared according to the 250 mL recipe above. A film of bacterial cells dried on a surface of a glass slide carrier. Four different lots of eight slides were prepared using two different bacteria: *Staphylococcus aureus* (ATCC 6538) and *Enterobacter aerogenes* (ATCC 13048). Five of the contaminated slides were exposed to the PAA test solution for either two minutes or five minutes at 18.0°C. After exposure, the neutralizing subculture medium (Letheen broth + 0.1% sodium thiosulfate + 0.01 % catalase) was added to each jar containing the glass slide carrier and the PAA test solution, and the solution was assayed for survivors. Three control slides from each lot were not exposed to a water solution and similarly treated with neutralizing subculture medium.

### Assay 3: AOAC Germicidal Efficacy - Use-Dilution Method

PAA test solutions were prepared according to the 2000 mL recipe above. A film of bacterial cells was dried on the surface of a lot of 30 stainless steel carrier items. Three different lots were prepared using three different bacteria: *Pseudomonas aeruginosa* (ATCC 15442), *Staphylococcus aureus* (ATCC 6538), and *Salmonella choleraesuis* (ATCC 10708). Each of the contaminated items was exposed to the PAA test solution for either 5 minutes or 10 minutes at room temperature (20.0°C). After exposure, the items were transferred to vessels containing neutralizing subculture medium (Letheen broth + 0.1% sodium thiosulfate + 0.01% catalase) and assayed for surviving bacteria. Appropriate purity, viability, carrier item sterility, neutralizing subculture medium sterility, viability, neutralization confirmation and carrier population controls also were performed.

### Assay 4: AOAC Sporicidal Efficacy

PAA test solutions were prepared according to the 250 mL recipe above. A suspension of *Bacillus stibtilis* (ATCC 19659) spores was dried on a lot of 30 Dacron sutures. A suspension of *Clostridium sporogenes* (ATCC 3584) was dried on a lot of 30 porcelain penicylinders. Each of the contaminated items was exposed to the PAA test solution for either 2 hours or 5.5 hours at room temperature (20.0°C). After exposure, the items were transferred to vessels containing neutralizing subculture medium (thiolglycollate medium + 0.1% sodium thiosulfate + 0.01% catalase) and assayed for surviving bacteria. Appropriate HCl, viability, purity, sterility, contaminated item quantitation, and neutralization controls also were performed.

### Results

Assay 1: The PAA test solution demonstrated > 99.999% reduction of either *Staphylococcus aureus* (ATCC 6538) or *Escherichia coli* (ATCC 11229) after only a 30 second exposure at 20.0°C.

Assay 2: The PAA test solution demonstrated > 99.9% reduction of either *Staphylococcus aureus* (ATCC 6538) or *Enterobacter aerogenes* (ATCC 13048) on glass slides following two-minute (or five minute) exposure at 18.0°C.

Assay 3: Five minute exposure of the contaminated stainless steel carrier items to the PAA test solution resulted in no demonstrated growth in any of 30 subculture tubes of any of *Pseudomonas aeruginosa* (ATCC 15442), *Staphylococcus aureus* (ATCC 6538), and *Salmonella choleraesuis* (ATCC 10708). One of 30 items exposed for ten minutes to the PAA solution did exhibit growth of *Pseudomonas aeruginosa* (ATCC 15442), however there was no demonstrated growth for any of the other bacteria in any of the ten minute exposure subculture tubes.

Assay 4: No demonstrated growth of *Bacillus subtilis* (ATCC 19659) in any of the 30 primary subcultures and no growth in any of the 30 secondary subcultures when tested using contaminated Dacron sutures exposed to the PAA solution for 2 hours or 5.5 hours at 20.0°C. Similarly, no demonstrated growth of *Clostridium sporogenes* (ATCC 3584) in any of the 30 primary subcultures and no growth in any of the 30 secondary subcultures when tested using contaminated porcelain penicylinders exposed to the PAA solution for 2 hours or 5.5 hours at 20.0°C.

### Conclusions

The 0.16% PAA solution generated using a three component formulation of S54V-MsAcT enzyme, propylene glycol diacetate (as ester substrate), and sodium percarbonate (as peroxide source), was highly effective in all four EPA approved disinfection/decontamination assays tested. The Germicidal and Detergent Sanitizing Action of Disinfectants test results show greater that 99.999% effectiveness against *Staphylococcus aureus* and *Escherichia coli.* The Standard Test Method for Efficacy of Sanitizers Recommended for Inanimate Non-Food Contact Surfaces test results show greater than 99.9% effectiveness against *Stapylococcus aureus* and *Enterobacter aerogenes.* The AOAC Use-Dilution Method test results showed 100% effectiveness against *Staphylococcus aureus*, *Salmonella choleraesius*, and 96.7% effectiveness against *Pseudomonas aercrginosa.* The Sporicidal Activity of Disinfectants (AOAC) assay demonstrated 100% efficacy of the PAA solution against *Bacillus subtilis* and *Clostridium sporogenes* spores.

### EXAMPLE 9

### A Stable "All-In-One" PAA Generating Composition With High Sporicidal Activity

This example illustrates the preparation, and stability over time, of an "All-In-One" composition of S54V-MsAcT enzyme and sodium percarbonate in the liquid ester substrate, propylene glycol diacetate. The example also illustrates the use of this composition to generate a 0.16 % by weight PAA solution.

### Materials and Methods

All-in-One Composition: To a tube of the dry formulation (containing 1.22 g of sodium percarbonate and 28.0 mg of S54V-MsAcT granules) prepared as described in Example 8 was added 1.8 mL of PGDA liquid.

Activation and Assay of All-in-One Composition: The All-in-One composition as prepared above was added to 250 mL of deionized water with stirring. Two additional PAA solutions were prepared according to the 250 mL and 2000 mL recipes described above in Example 8.

At t = 20 minutes, the pH of each of these three solutions was measured and found to be 10.3. Also at t = 20 minutes, a 20 µL aliquot was taken from each of the three solutions and added to 980 µL of 125 mM sodium citrate, pH 5.0. These three diluted samples were then assayed for PAA concentration using the ABTS assay described above.

Stability Study: Two additional All-in-One composition samples were prepared. One was maintained for 4 days before being activated in 250 mL water and assaying for PAA concentration. The other was maintained for 33 days at room temperature before being activated in 250 mL water and assaying for PAA concentration. For comparison, samples were prepared as in Example 8 with the dry formulation components maintained separately from the liquid PGDA component for the same 4 day and 33 day periods at room temperature before being activated in water.

Variable PAA Generating Compositions: A set of five All-in-One compositions with variable amounts of sodium percarbonate were prepared to demonstrate the ability to vary the concentration of PAA generated over a range between about 60% and 160% of the standard composition. The five All-in-One composition samples were made up according to amounts listed in Table 5.

**TABLE 5**

| Sample # | Sodium percarbonate (g) | Enzyme (mg) | PGDA (mL) |
|---|---|---|---|
| 1 | 0.768 | 30.7 | 1.8 |
| 2 | 1.003 | 30.9 | 1.8 |
| 3 | 1.251 | 29.6 | 1.8 |
| 4 | 1.501 | 28.7 | 1.8 |
| 5 | 2.009 | 30.5 | 1.8 |

These five samples were then added to 250 mL deionized water with stirring at ambient temperature (20°C). Sample aliquots for assay were taken at 10, 20, and 30 minutes. PAA concentration was assayed using the ABTS assay in a microtiter plate.

Sporicidal Efficacy Assay: An All-In-One composition was prepared containing 1.37 g sodium percarbonate, 33.30 mg S54V-MsAcT, mixed with 1.80 mL PGDA in a 15.0 mL conical tube. The composition was added to sterile deionized water with stirring for 20 minutes. 10 mL of the resulting PAA test solution was placed into each of four 25 x 150 mm tubes. These tubes were placed in a 20°C incubator and allowed to come to temperature. Using sterile forceps, five penicylinders, contaminated with *B. subtilis,* or five suture loops, contaminated with *B. subtilis,* were added to each tube. After the addition of all of the cylinders or sutures to each solution, a period of five minutes was allowed to make time for later removal from the tubes. After a contact time of one hour at 20°C, the penicylinders or sutures were removed, each to a separate tube of 10 mL fluid thioglycollate medium (Difco Cat. # 225650) using bent sterile plastic needles (tips bent aseptically while inside the bag into a hook). After completing transfer to the primary tube of thiolglycollate medium, the cylinders and sutures were transferred to a fresh tube of thioglycollate medium (10 mL) and incubated for 21 days at 37°C. If no growth was observed after 21 days, the tubes were heat-shocked at 80°C and re-incubated for an additional 72 hours at 37 °C. Nineteen to twenty replicates were run.

### Results

The All-in-One composition when activated in 250 mL of water with stirring for 20 minutes generated a solution with 0.18% peracid. The 250 mL and 2000 mL recipes prepared in the same assay yielded solutions with 0.17% and 0.17% peracid, respectively.

The solutions prepared with the All-in-One composition maintained for 4 days and 33 days at room temperature generated 100% of the expected concentration of peracid. Similarly, the solutions prepared using the separate dry formulation and liquid components yielded essentially 100% of their expected PAA concentration.

The five All-in-One composition samples prepared with varying amounts sodium percarbonate yielded a range of PAA concentrations from 0.11% to 0.33% at the 20 minute time point that constitutes the standard usage conditions. Thus, the All-in-One composition can be "tuned" to provide a desired PAA concentration within a specified range. In this particular example, the concentration of PAA generated ranged from 19-51 mM, which was 28.5 - 76.5 mM H₂O₂ (Na₂CO₃-1.5H₂O₂). The maximum conversion rate of H₂O₂ was 61.7% observed for sample 5 at t = 30 minutes. Based on this, a desired amount of PAA can be achieved by adjusting the percarbonate level or the PGDA level, or both.

The All-in-One composition made with 1.37 g sodium percarbonate, 33.30 mg S54V-MsAcT, mixed with 1.80 mL PGDA in 250 mL water exhibited no failures in sporicidal efficacy for the *B. subtilis* contaminated sutures and penicylinders.

### Conclusions

An "All-in-One" composition containing all three components for enzymatic PAA generation in a single tube can be used to generate an effective PAA decontamination solution (0.16%) equally as well as formulations where the dry components are stored separate from the liquid PGDA ester substrate. Remarkably, the All-in-One composition exhibited stability with 0% loss of performance in PAA generation even after 33 days at room temperature before activating in water. Moreover, the PAA solutions prepared using the "All-in-One" composition showed high levels of sporicidal efficacy equivalent to the separate dry and liquid component formulations when assayed on contaminated sutures and porcelain penicylinders.

Furthermore, it was found that the All-in-One composition can be "tuned" by varying the amount of sodium percarbonate used so as to generate PAA concentrations ranging from about 0.11% to 0.33%.

### EXAMPLE 10

### Use of Water Soluble Sachet for Delivery of An All-In-One PAA Generating Composition

This example illustrates the preparation and use of an All-In-One composition for PAA generation formulated in a water-soluble sachet.

### Materials and Methods

Water soluble polyvinyl alcohol (PVA) sachets were obtained from Solupak Ltd. (Manchester. UK). Ten of these sachets were prepared for use with the All-In-One composition by filling each with 7.5 g of PGDA, 4.8 g of sodium percarbonate and 110 mg of the acyl transferase S54V-MsAcT. Each sachet was then sealed using a heat sealer.

Sachets were shipped from the UK to the US, resulting in a transit time of 7 days before use. Upon receipt, a sachet was dissolved in 2000 mL of purified water (MilliQ) at 21 °C. The pH and PAA concentration of the 2000 mL solution was measured at selected time points over 50 minute time course. PAA was assayed using the ABTS in citrate assay, as described above.

### Results

The sachet dissolved in the 2000 mL solution as expected delivering the All-In-One composition and resulting in the following PAA concentrations and pH values listed in Table 6.

**TABLE 6**

| **Time (min)** | **PAA (wt. %)** | **pH** |
|---|---|---|
| 0.00 | 0.02 | 5.20 |
| 5.00 | 0.02 | 10.10 |
| 10.00 | 0.04 | 10.19 |
| 15.00 | 0.08 | 9.78 |
| 20.00 | 0.12 | 9.37 |
| 25.00 | 0.14 | 8.97 |
| 30.00 | 0.16 | 8.62 |
| 50.00 | 0.18 | 8.12 |

As shown above, the sachet delivery system performed as scaled. It generated the targeted PAA concentration and gave a typical pH profile, with a pH of 8.6 being generated at 20 minutes (the proposed dissolution time), which is a pH range generally considered as non-corrosive.

### Conclusions

The All-In-One composition is stable and can be delivered to solution effectively in a water soluble polyvinyl alcohol sachet. The resulting PAA concentrations and pH values of the solution after 30 minutes that are equivalent to those observed without the sachet.

All patents and publications mentioned in the specification are indicative of the levels of those skilled in the art to which the invention pertains. inherent therein. The compositions and methods described herein are representative of preferred embodiments, are exemplary, and are not intended as limitations on the scope of the invention. It is readily apparent to one skilled in the art that varying substitutions and modifications may be made to the invention disclosed herein without departing from the scope and spirit of the invention.

The invention illustratively described herein suitably may be practiced in the absence of any element or elements, limitation or limitations which is not specifically disclosed herein. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed. Thus, it should be understood that although the present invention has been specifically disclosed by preferred embodiments and optional features, modification and variation of the concepts herein disclosed may be resorted to by those skilled in the art, and that such modifications and variations are considered to be within the scope of this invention as defined by the appended claims.

The invention has been described broadly and generically herein. Each of the narrower species and sub generic groupings falling within the generic disclosure also form part of the invention. This includes the generic description of the invention with a proviso or negative limitation removing any subject matter from the genus, regardless of whether or not the excised material is specifically recited herein.

## Claims

1. An all-in-one composition comprising a perhydrolase enzyme, a hydrogen peroxide source, and an ester substrate, wherein said composition generates a peracid when mixed with water, and wherein said ester comprises less than 5% by weight of water, and wherein the ester substrate is a liquid and the perhydrolase enzyme and hydrogen peroxide source are dry solids suspended in the liquid, and wherein the ester substrate is selected from the group consisting of: methyl acetate, ethyl acetate, propyl acetate, triacetin (1,3-diacetyloxypropan-2-yl acetate) and propylene glycol diacetate.

2. The composition of claim 1, wherein said ester comprises less than 1%, less than 0.5% or less than 0.1 % by weight of water.

3. The composition of claim 1, wherein the peracid is selected from peracetic acid, pernonanoic acid, perpropionic acid, perbutanoic acid, perpentanoic acid, and perhexanoic acid.

4. The composition of claim 3, wherein the peracid is peracetic acid.

5. The composition of claim 1, wherein the ester substrate is propylene glycol diacetate.

6. The composition of claim 1, wherein the perhydrolase enzyme comprises the amino acid sequence set forth in SEQ ID NO: 1.

7. The composition of claim 1, wherein the perhydrolase enzyme is the S54V variant of SEQ ID NO: 1.

8. The composition of claim 1, wherein the hydrogen peroxide source is a hydrogen peroxide generating compound selected from sodium percarbonate, sodium perborate, and urea hydrogen peroxide.

9. The composition of claim 8, wherein the hydrogen peroxide source is sodium percarbonate.

10. The composition of claim 1, wherein the composition comprises 0.001% to 1% by weight perhydrolase enzyme, 35% to 45% by weight sodium percarbonate, and 55% to 65% by weight propylene glycol diacetate.

11. The composition of claim 1, wherein the hydrogen peroxide source is an enzymatic hydrogen peroxide generation system comprising an oxidase and its substrate.

12. The composition of claim 11, wherein the oxidase is selected from glucose oxidase, sorbitol oxidase, hexose oxidase, choline oxidase, alcohol oxidase, glycerol oxidase, cholesterol oxidase, pyranose oxidase, carboxyalcohol oxidase, L-amino acid oxidase, glycine oxidase, pyruvate oxidase, glutamate oxidase, sarcosine oxidase, lysine oxidase, lactate oxidase, vanillyl oxidase, glycolate oxidase, galactose oxidase, uricase, oxalate oxidase, and xanthine oxidase.

13. The composition of claim 1, further comprising at least one additional enzyme selected from proteases, cellulases, amylases, and microbial cell wall degrading enzymes.

14. The composition of claim 1, wherein the composition further comprises at least one surfactant.

15. The composition of claim 1, wherein the composition retains peracid generating activity for at least 7 days after being stored at an ambient temperature from 18°C to 25°C.

16. A method for preparing a decontamination solution comprising adding the composition of any of claims 1 to 15 to water, wherein said composition has been prior stored at ambient temperature for at least 7 days, and mixing for at least 20 minutes.

17. A method for decontamination comprising:
(a) providing a composition according to any of claims 1 to 15, wherein said composition has been prior stored at ambient temperature for at least 7 days;
(b) adding said composition to water and mixing, thereby generating an aqueous peracid solution;
(c) exposing an item comprising a contaminant to said solution.

18. The method of claim 17, wherein the composition is contained in a water soluble container.

19. The method of claim 18, wherein the container is a sachet made of polyvinyl alcohol film.

20. The method of claim 17, wherein the water is sterilized.

21. The method of claim 17, wherein said exposing comprises exposing said item to said solution at high temperature.

22. The method of claim 17, wherein said contaminant comprises a toxin selected from botulinum toxin, anthracis toxin, ricin, scombroid toxin, ciguatoxin, tetrodotoxin, mycotoxins, and any combination thereof.

23. The method of claim 17, wherein said contaminant comprises a pathogen selected from bacteria, viruses, fungi, parasites, prions, and any combination thereof.

24. The method of claim 23, wherein said pathogen is selected *Bacillus spp., B. anthracis, Clostridium spp., C. botulinum, C. perfringens, Listeria spp., Staphylococcus spp., Streptococcus spp., Salmonella spp., Shigella ssp., E. coli, Yersinia spp., Y. pestis, Francisella spp., F. tularensis, Camplyobacter ssp., Vibrio spp., Brucella spp., Cryptosporidium spp., Giardia spp., Cyclospora spp., and Trichinella spp.*

25. The method of claim 17, wherein said item is selected from hard surfaces, fabrics, food, feed, apparel, rugs, carpets, textiles, medical instruments, and veterinary instruments.

26. The method of claim 17, wherein said ambient temperature is from 18°C to 25°C.

27. A system comprising a water soluble container comprising a stable composition according any of claims 1 to 15.

28. The system of claim 27, wherein said container is a sachet, ampoule, capsule, or sphere.

29. The system of claim 27, wherein the water soluble container is made of a water soluble polymer material polyvinyl alcohol, polyethylene oxides, cellulose derived water soluble carbohydrates, and polylactides.

30. A method for preparing a decontamination solution comprising adding system of claim 27 to water and mixing.

31. A kit for decontamination comprising the stable composition of any of claims 1 to 15 in packaging.

32. The kit of claim 31, wherein the composition is in a water soluble container.

33. The kit of claim 32, wherein the water soluble container is a sachet, ampoule, capsule, or sphere.

34. The kit of claim 32, wherein the water soluble container is made of a water soluble polymer material selected from polyvinyl alcohol, polyethylene oxides, cellulose derived water soluble carbohydrates, and polylactides.

35. The kit of claim 31, further comprising an empty mixing container for mixing said composition with water.

36. The kit of claim 31, further comprising water in a sealed container, wherein the water is sterilized.

## Patentansprüche

1. Komplettzusammensetzung umfassend ein Perhydrolaseenzym, eine Wasserstoffperoxidquelle und ein Estersubstrat, wobei die Zusammensetzung eine Persäure erzeugt, wenn sie mit Wasser gemischt wird, und wobei der Ester weniger als 5 Gew.-% Wasser umfasst, und wobei das Estersubstrat eine Flüssigkeit ist und das Perhydrolaseenzym und die Wasserstoffperoxidquelle trockene Feststoffe sind, die in der Flüssigkeit suspendiert sind, und wobei das Estersubstrat aus der Gruppe ausgewählt ist bestehend aus: Methylacetat, Ethylacetat, Propylacetat, Triacetin (1,3-Diacetyloxypropan-2-ylacetat) und Propylenglycoldiacetat.

2. Zusammensetzung nach Anspruch 1, wobei der Ester weniger als 1, weniger als 0,5 oder weniger als 0,1 Gew.-% Wasser umfasst.

3. Zusammensetzung nach Anspruch 1, wobei die Persäure unter Peressigsäure, Pernonansäure, Perpropionsäure, Perbuttersäure, Perpentansäure und Perhexansäure ausgewählt wird.

4. Zusammensetzung nach Anspruch 3, wobei die Persäure Peressigsäure ist.

5. Zusammensetzung nach Anspruch 1, wobei das Estersubstrat Propylenglycoldiacetat ist.

6. Zusammensetzung nach Anspruch 1, wobei das Perhydrolaseenzym die in SEQ ID NO:1 aufgeführte Aminosäuresequenz umfasst.

7. Zusammensetzung nach Anspruch 1, wobei das Perhydrolaseenzym die S54V-Variante von SEQ ID NO:1 ist.

8. Zusammensetzung nach Anspruch 1, wobei die Wasserstoffperoxidquelle eine Wasserstoffperoxid erzeugende Verbindung ist ausgewählt unter Natriumcarbonat, Natriumperborate und Harnstoffwasserstoffperoxid.

9. Zusammensetzung nach Anspruch 8, wobei die Wasserstoffperoxidquelle Natriumpercarbonat ist.

10. Zusammensetzung nach Anspruch 1, wobei die Zusammensetzung 0,001 bis 1 Gew.-% Perhydrolaseenzym, 35 bis 45 Gew.-% Natriumpercarbonat und 55 bis 65 Gew.-% Propylenglycoldiacetat umfasst.

11. Zusammensetzung nach Anspruch 1, wobei die Wasserstoffperoxidquelle ein enzymatisches Wasserstoffperoxid erzeugendes System ist, das eine Oxidase und ihr Substrat umfasst.

12. Zusammensetzung nach Anspruch 11, wobei die Oxidase unter Glucoseoxidase, Sorbitoxidase, Hexoseoxidase, Cholinoxidase, Alkoholoxidase, Glycerinoxidase, Cholesterinoxidase, Pyranoseoxidase, Carboxyalkoholoxidase, L-Aminosäureoxidase, Glycinoxidase, Pyruvatoxidase, Glutamatoxidase, Sarcosinoxidase, Lysinoxidase, Lactatoxidase, Vanillyloxidase, Glycolatoxidase, Galactoseoxidase, Uricase, Oxalatoxidase und Xanthinoxidase ausgewählt wird.

13. Zusammensetzung nach Anspruch 1, ferner mindestens ein zusätzliches Enzym umfassend ausgewählt unter Proteasen, Cellulasen, Amylasen und mikrobiellen Zellwand abbauenden Enzymen.

14. Zusammensetzung nach Anspruch 1, wobei die Zusammensetzung ferner mindestens ein Tensid umfasst.

15. Zusammensetzung nach Anspruch 1, wobei die Zusammensetzung Persäure erzeugende Aktivität mindestens 7 Tage lang bei Lagerung bei Umgebungstemperatur von 18 °C bis 25 °C beibehält.

16. Verfahren für die Herstellung einer Dekontaminationslösung, umfassend das Zusetzen der Zusammensetzung nach einem der Ansprüche 1 bis 15 zu Wasser, wobei die Zusammensetzung vorher bei Umgebungstemperatur mindestens 7 Tage lang gelagert worden ist, und das Mischen mindestens 20 Minuten lang dauert.

17. Verfahren zum Dekontaminieren, umfassend:
(a) das Bereitstellen einer Zusammensetzung nach einem der Ansprüche 1 bis 15, wobei die Zusammensetzung vorher bei Umgebungstemperatur mindestens 7 Tage lang gelagert worden ist;
(b) das Zugeben der Zusammensetzung zu Wasser und Mischen, wodurch eine wässrige Persäurelösung erzeugt wird
(c) das Aussetzen eines Artikels, das einen Kontaminationsstoff umfasst, der Lösung.

18. Verfahren nach Anspruch 17, wobei die Zusammensetzung in einem wasserlöslichen Behälter enthalten ist.

19. Verfahren nach Anspruch 18, wobei der Behälter ein aus Polyvinylalkoholfolie hergestellter Beutel ist.

20. Verfahren nach Anspruch 17, wobei das Wasser sterilisiert ist.

21. Verfahren nach Anspruch 17, wobei das Aussetzen das Aussetzen des Artikels der Lösung bei hoher Temperatur umfasst.

22. Verfahren nach Anspruch 17, wobei der Kontaminationsstoff ein Toxin umfasst ausgewählt unter Botulinumtoxin, Anthraxtoxin, Ricin, Scrombroidtoxin, Ciguatoxin, Tetrodotoxin, Mycotoxinen und irgendeiner Kombination davon.

23. Verfahren nach Anspruch 17, wobei der Kontaminationsstoff ein Pathogen umfasst ausgewählt unter Bakterien, Viren, Pilzen, Parasiten, Prionen und irgendeiner Kombination davon.

24. Verfahren nach Anspruch 23, wobei das Pathogen ausgewählt ist unter *Bacillus spp., B. anthracis*, *Clostridium spp, C. botulinum*, *C. perfringens, Listeria spp., Staphylococcus spp., Streptococcus spp., Salmonella spp., Shigella ssp., E. coli, Yersinia spp., Y. pestis, Francisella spp., F. tularensis*, *Camphylobacter ssp., Vibrio spp., Brucella spp., Cryptosporidium spp., Giardia spp., Cyclospora spp.* und *Trichinella spp.*

25. Verfahren nach Anspruch 17, wobei der Artikel unter harten Oberflächen. Textilstoffen, Nahrungsmitteln, Futter, Kleidungsstücken, Vorlegern, Teppichen, Textilien, medizinischen Instrumenten und tierärztlichen Instrumenten ausgewählt wird.

26. Verfahren nach Anspruch 17, wobei die Umgebungstemperatur 18 °C bis 25°C beträgt.

27. System umfassend einen wasserlöslichen Behälter, der eine beständige Zusammensetzung nach einem der Ansprüche 1 bis 15 umfasst.

28. System nach Anspruch 27, wobei der Behälter ein Beutel, eine Ampulle, Kapsel oder Kugel ist.

29. System nach Anspruch 27, wobei der wasserlösliche Behälter aus wasserlöslichem Polymermaterial Polyvinylalkohol, Polyethylenoxiden, von wasserlöslichen Kohlehydraten aus Cellulose und Polylactiden hergestellt ist.

30. Verfahren für die Herstellung einer Dekontaminierungslösung, umfassend das Zusetzen des Systems nach Anspruch 27 zu Wasser und Mischen.

31. Kit zum Dekontaminieren, umfassend die beständige Zusammensetzung nach einem der Ansprüche 1 bis 15 in Verpackung.

32. Kit nach Anspruch 31, wobei die Zusammensetzung ein wasserlöslicher Behälter ist.

33. Kit nach Anspruch 32, wobei der wasserlösliche Behälter ein Beutel, eine Ampulle, Kapsel oder Kugel ist.

34. Kit nach Anspruch 32, wobei der wasserlösliche Behälter aus einem wasserlöslichen Polymermaterial ausgewählt unter Polyvinylalkohol, Polyethylenoxiden, von wasserlöslichen Kohlehydraten aus Cellulose und Polylactiden hergestellt ist.

35. Kit nach Anspruch 31, ferner einen leeren Mischbehälter für das Mischen der Zusammensetzung mit Wasser umfassend.

36. Kit nach Anspruch 31, ferner Wasser in einem versiegelten Behälter umfassend, wobei das Wasser sterilisiert ist.

## Revendications

1. Composition tout-en-un comprenant une enzyme perhydrolase, une source de peroxyde d'hydrogène, et un substrat de type ester, ladite composition générant un peracide lorsqu'elle est mélangée avec de l'eau, et ledit ester comprenant moins de 5 % en poids d'eau, et le substrat de type ester étant un liquide et l'enzyme perhydrolase et la source de peroxyde d'hydrogène étant des matières solides sèches en suspension dans le liquide, et le substrat de type ester étant sélectionné dans le groupe constitué du: acétate de méthyle, acétate d'éthyle, acétate de propyle, de la (acétate de 1,3-diacétyloxypropan-2-yle) triacétine et du diacétate de propylène glycol.

2. Composition selon la revendication 1, ledit ester comprenant moins de 1 %, moins de 0,5 % ou moins de 0,1 % en poids d'eau.

3. Composition selon la revendication 1, le peracide étant sélectionné parmi l'acide peracétique, l'acide pernonanoïque, l'acide perpropionique, l'acide perbutanoïque, l'acide perpentanoïque, et l'acide perhexanoïque.

4. Composition selon la revendication 3, le peracide étant l'acide peracétique.

5. Composition selon la revendication 1, le substrat de type ester étant le diacétate de propylène glycol.

6. Composition selon la revendication 1, l'enzyme perhydrolase comprenant la séquence d'acides aminés exposée dans SEQ ID n°: 1.

7. Composition selon la revendication 1, l'enzyme perhydrolase étant le variant S54V de SEQ ID n°: 1.

8. Composition selon la revendication 1, la source de peroxyde d'hydrogène étant un composé générant du peroxyde d'hydrogène sélectionné parmi le percarbonate de sodium, le perborate de sodium, et l'hydrogénoperoxyde d'urée.

9. Composition selon la revendication 8, la source de peroxyde d'hydrogène étant le percarbonate de sodium.

10. Composition selon la revendication 1, la composition comprenant 0,001 % à 1 % en poids d'enzyme perhydrolase, 35 % à 45 % en poids de percarbonate de sodium, et 55 % à 65 % en poids de diacétate de propylène glycol.

11. Composition selon la revendication 1, la source de peroxyde d'hydrogène étant un système de génération enzymatique de peroxyde d'hydrogène comprenant une oxydase et son substrat.

12. Composition selon la revendication 11, l'oxydase étant sélectionnée parmi la glucose oxydase, la sorbitol oxydase, l'hexose oxydase, la choline oxydase, l'alcool oxydase, la glycérol oxydase, la cholestérol oxydase, la pyranose oxydase, la carboxyalcool oxydase, l'acide L-amino oxydase, la glycine oxydase, la pyruvate oxydase, la glutamate oxydase, la sarcosine oxydase, la lysine oxydase, la lactate oxydase, la vanillyl oxydase, la glycolate oxydase, la galactose oxydase, l'uricase, oxalate oxydase, et la xanthine oxydase.

13. Composition selon la revendication 1, comprenant en outre au moins une enzyme supplémentaire sélectionnée parmi les protéases, les cellulases, les amylases, et les enzymes de dégradation de la paroi cellulaire microbienne.

14. Composition selon la revendication 1, la composition comprenant en outre au moins un tensioactif.

15. Composition selon la revendication 1, la composition conservant l'activité de génération de peracide durant au moins 7 jours après avoir été stockée à une température ambiante allant de 18°C à 25°C.

16. Procédé de préparation d'une solution de décontamination comprenant l'addition de la composition de n'importe laquelle des revendications 1 à 15 à de l'eau, ladite composition ayant été stockée au préalable à la température ambiante durant au moins 7 jours, et le mélange durant au moins 20 minutes.

17. Procédé de décontamination comprenant:
(a) la fourniture d'une composition selon l'une quelconque des revendications 1 à 15, ladite composition ayant été stockée au préalable à la température ambiante durant au moins 7 jours;
(b) l'addition de ladite composition à de l'eau et le mélange, générant ainsi une solution aqueuse de peracide;
(c) l'exposition d'un article comprenant un contaminant à ladite solution.

18. Procédé selon la revendication 17, la composition étant contenue dans un récipient soluble dans l'eau.

19. Procédé selon la revendication 18, le récipient étant un sachet constitué d'un film de poly(alcool de vinyle).

20. Procédé selon la revendication 17, l'eau étant stérilisée.

21. Procédé selon la revendication 17, ladite exposition comprenant l'exposition dudit article à ladite solution à température élevée.

22. Procédé selon la revendication 17, ledit contaminant comprenant une toxine sélectionnée parmi la toxine botulique, la toxine de Bacillus anthracis, le ricin, la toxine scombroïde, la toxine de ciguë, la tétrodotoxine, les mycotoxines, et n'importe quelle combinaison de celles-ci.

23. Procédé selon la revendication 17, ledit contaminant comprenant un pathogène sélectionné parmi les bactéries, les virus, les champignons, les parasites, les prions, et n'importe quelle combinaison de ceux-ci.

24. Procédé selon la revendication 23, ledit pathogène étant sélectionné parmi *Bacillus spp., B. anthracis*, *Clostridium spp., C. botulinum, C. perfringens, Listeria spp., Staphylococcus spp., Streptococcus spp., Salmonella spp., Shigella ssp, E. coli, Yersinia spp., Y. pestis, Francisella spp., F. tularensis*, *Campylobacter ssp., Vibrio spp., Brarcella spp., Cryptosporidium spp., Giardia spp., Cyclospora spp.,* et *Trichinella spp.*

25. Procédé selon la revendication 17, ledit article étant sélectionné parmi les surfaces dures, les tissus, les aliments, les aliments pour animaux, les vêtements, les paillassons, les tapis, les textiles, les instruments médicaux, et les instruments à usage vétérinaire.

26. Procédé selon la revendication 17, ladite température ambiante étant de 18°C à 25°C.

27. Système comprenant un récipient soluble dans l'eau comprenant une composition stable selon l'une quelconque des revendications 1 à 15.

28. Système selon la revendication 27, ledit récipient étant un sachet, une ampoule, une capsule, ou une sphère.

29. Système selon la revendication 27, le récipient soluble dans l'eau étant fabriqué à partir d'un matériau polymère soluble dans l'eau de poly(alcool de vinyle), de poly(oxydes d'éthylène), d'hydrates de carbone dérivés de cellulose solubles dans l'eau, et des polylactides.

30. Procédé de préparation d'une solution de décontamination comprenant l'addition du système selon la revendication 27 à de l'eau et le mélange.

31. Trousse de décontamination comprenant la composition stable selon l'une quelconque des revendications 1 à 15 dans un emballage.

32. Trousse selon la revendication 31, la composition étant un récipient soluble dans l'eau.

33. Trousse selon la revendication 32, le récipient soluble dans l'eau étant un sachet, une ampoule, une capsule, ou une sphère.

34. Trousse selon la revendication 32, le récipient soluble dans l'eau étant fabriqué à partir d'un matériau polymère soluble dans l'eau sélectionné parmi le poly(alcool de vinyle), les poly(oxydes d'éthylène), les hydrates de carbone dérivés de cellulose solubles dans l'eau, et les polylactides.

35. Trousse selon la revendication 31, comprenant en outre un récipient vide de mélange pour mélanger ladite composition avec de l'eau.

36. Trousse selon la revendication 31, comprenant en outre de l'eau dans un récipient hermétiquement fermé, l'eau étant stérilisée.
